# EUROPEAN PATENT SPECIFICATION

(11) **EP 2 555 795 B1**
(45) Date of publication and mention of the grant of the patent: **13.07.2016**
(21) Application number: 11715069.8
(22) Date of filing: 08.04.2011
(51) Int. Cl.: A61K 39/00, A61K 39/145, C12N 9/24, A61K 39/12

(54) **RECOMBINANT MULTIMERIC INFLUENZA PROTEINS**
REKOMBINANTE MULTIMERE INFLUENZAPROTEINE
PROTÉINES DE LA GRIPPE MULTIMÈRES RECOMBINANTES

(30) Priority: 09.04.2010 EP 10159552
(43) Date of publication of application: 13.02.2013
(73) Proprietor: Universiteit Utrecht Holding B.V., 3584 CM Utrecht (NL)
(72) Inventor: ROTTIER, Petrus, Josephus, Marie, NL-3737 RG Groenekan (NL); BOSCH, Berend, Jan, NL-3994 TP Houten (NL); DE HAAN, Cornelis, Alexander, Maria, NL-3993 RH Houten (NL)
(74) Representative: Intervet International B.V.
(86) International application number: PCT/NL2011/050234
(87) International publication number: WO 2011/126370

(56) References cited:
- WO-A1-2009/133463
- WO-A2-02/074795
- WEI CHIH-JEN ET AL: "Comparative efficacy of neutralizing antibodies elicited by recombinant hemagglutinin proteins from avian H5N1 influenza virus", JOURNAL OF VIROLOGY, THE AMERICAN SOCIETY FOR MICROBIOLOGY, US, vol. 82, no. 13, 1 July 2008 (2008-07-01), pages 6200-6208, XP009138042, ISSN: 0022-538X
- DE FILETTE MARINA ET AL: "An influenza A vaccine based on tetrameric ectodomain of matrix protein 2", JOURNAL OF BIOLOGICAL CHEMISTRY, AMERICAN SOCIETY FOR BIOCHEMISTRY AND MOLECULAR BIOLOGY, INC, US LNKD- DOI:10.1074/JBC.M800650200, vol. 283, no. 17, 25 April 2008 (2008-04-25), pages 11382-11387, XP002518513, ISSN: 0021-9258 [retrieved on 2008-02-05]
- TERPE K: "Overview of tag protein fusions: from molecular and biochemical fundamentals to commercial systems", APPLIED MICROBIOLOGY AND BIOTECHNOLOGY, SPRINGER, BERLIN, DE, vol. 60, 1 January 2003 (2003-01-01), pages 523-533, XP009091798, ISSN: 1432-0614
- ZWICKER N ET AL: "Strep-tag(R) II for one-step affinity purification of active bHLHzip domain of human c-Myc", BIOTECHNIQUES, INFORMA LIFE SCIENCES PUBLISHING, WESTBOROUGH, MA, US, vol. 27, no. 2, 1 August 1999 (1999-08-01) , pages 368-370,372, XP001525275, ISSN: 0736-6205
- HERFST SANDER ET AL: "Immunization of Syrian golden hamsters with F subunit vaccine of human metapneumovirus induces protection against challenge with homologous or heterologous strains", JOURNAL OF GENERAL VIROLOGY, SOCIETY FOR GENERAL MICROBIOLOGY, SPENCERS WOOD, GB, vol. 88, no. 10, 1 October 2007 (2007-10-01), pages 2702-2709, XP009138051, ISSN: 0022-1317
- STILLS HAROLD F JR: "Adjuvants and antibody production: dispelling the myths associated with Freund's complete and other adjuvants", ILAR JOURNAL, INSTITUTE FOR LABORATORY ANIMAL RESEARCH, US, vol. 46, no. 3, 1 January 2005 (2005-01-01), pages 280-293, XP001539142, ISSN: 1084-2020
- LEENAARS P P A M ET AL: "Evaluation of several adjuvants as alternatives to the use of Freund's adjuvant in rabbits", VETERINARY IMMUNOLOGY AND IMMUNOPATHOLOGY, AMSTERDAM, NL LNKD- DOI:10.1016/0165-2427(94)90022-1, vol. 40, no. 3, 1 March 1994 (1994-03-01), pages 225-241, XP023690027, ISSN: 0165-2427 [retrieved on 1994-03-01]
- CORNELISSEN LISETTE A H M ET AL: "A Single Immunization with Soluble Recombinant Trimeric Hemagglutinin Protects Chickens against Highly Pathogenic Avian Influenza Virus H5N1", PLOS ONE, PUBLIC LIBRARY OF SCIENCE, SAN FRANCISCO, CA, US, vol. 5, no. 5, 1 May 2010 (2010-05-01), pages E10645-1, XP009138059, ISSN: 1932-6203
- DE VRIES ROBERT P ET AL: "The influenza A virus hemagglutinin glycosylation state affects receptor-binding specificity", VIROLOGY, ACADEMIC PRESS,ORLANDO, US, vol. 403, no. 1, 1 July 2010 (2010-07-01), pages 17-25, XP009138137, ISSN: 0042-6822, DOI: DOI:10.1016/J.VIROL.2010.03.047

## Description

The invention relates to the field of virology, more specifically the invention relates to the field of influenza virus vaccines.

Influenza is an infectious disease that is caused by three types of influenza viruses, types A, B and C, which belong to the family of Orthomyxoviridae. Influenza viruses are RNA viruses consisting of seven negative single-stranded RNA-segments encoding nine proteins (influenza C), or eight negative single-stranded RNA-segments encoding eleven proteins (influenza A and B) (Chen and Deng, 2009). Birds are thought to be the natural host for all influenza A viruses, but many subtypes may also infect mammals, including humans. Symptoms of influenza include fever, headache, chills, muscle pains and soar throat, symptoms comparable with the common cold. However, influenza can lead to life-threatening complications, such as pneumonia, and death in high-risk groups such as young children, the elderly, and immune compromised or chronically ill individuals. In the past, influenza pandemics, such as the 1918/1919 Spanish Flu pandemic (H1N1 subtype) and the 1968/1969 Hong Kong Flu (H3N2 subtype), have been responsible for the death of millions of people (Khanna et al., 2008).

The influenza viral surface proteins hemagglutinin (HA) and neuraminidase (NA) are the basis for serologically different influenza subtypes. Currently 16 HA (H1-H16) and 9 NA (N1-N9) serotypes have been identified, which are used to classify influenza viruses (e.g. H3N2). Antigenic drift, due to one or more mutations causing amino acid substitution(s), may cause minor antigenic changes in HA and NA, resulting in the escape of immunity of a host. Additionally, a process called antigenic shift results in the formation of new virus subtypes (reassortants) through combination of HA and NA from different influenza virus subtypes. These alterations in HA and/or NA may in part be a result of selection pressure (Chen and Deng, 2009).

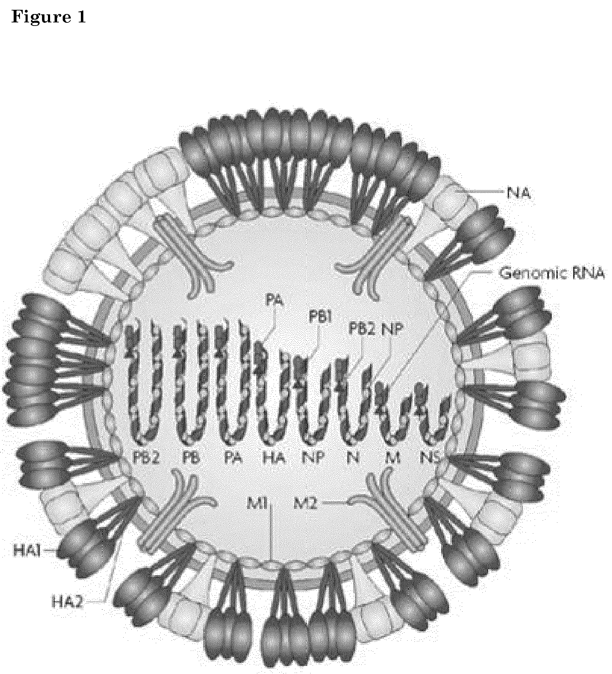

HA is present in the viral membrane. It must be cleaved by host proteases to yield two polypeptides, HA1 and HA2, in order to be infectious. Following cleavage, the newly exposed N-terminal of the HA2 polypeptide then acts to mediate fusion of the viral with the host cell membrane, which allows the virus to initiate infection of the host cell (Skehel and Wiley, 2000). HA is a homotrimer composed of three structurally distinct regions, a globular head consisting of anti-parallel beta-sheets which contains the receptor binding site and the HA1/HA2 cleavage site, a triple-stranded, coiled-coil, alpha-helical stalk, and a globular foot consisting of anti-parallel beta-sheets. In figure 1 a schematic representation of an influenza virus is shown. Each monomer consists of a HA polypeptide with the HA1 and HA2 regions linked by disulphide bonds (Skehel and Wiley, 2000; Stevens et al., 2006).

NA catalyses the hydrolysis of terminal sialic acid residues of glycoproteins of the host cell, thereby preventing binding of HA to these proteins. NA thus facilitates release of the virus from a cell and hence spreading of the virus. NA is a homotetramer, anchored to the viral membrane by its N-terminus. In figure 1 a schematic representation of an influenza virus is shown. Each monomer is composed of six topologically identical beta-sheets arranged in a propeller formation, which form the head of the tetrameric protein that is attached to a stalk region. Enzymatic activity is located in each monomer (Colman 1994).

Influenza virus infections are most prevalent in winter and seasonal influenza vaccines are developed each year. These vaccines contain two influenza A viruses (H1N1 and H3N2) and one influenza B virus (Khanna et al., 2008). New compositions need to be developed each year to protect against the predicted circulating strains. Even though they may have high homology, a specific vaccine may not protect against different strains from the same influenza A subtype. In addition to seasonal human influenza vaccines, several influenza vaccines for treatment of animals exist, such as vaccines against avian H5N1 and H9N2 influenza infection in poultry and H1N1 and H3N2 influenza infection in pigs. Currently available influenza vaccines are either live attenuated or inactivated influenza viruses which are generally produced by growing the virus in embryonized chicken eggs. Several disadvantages of this production method exist including the often high-level biocontainment facilities required to manage influenza viruses, the inability to obtain high yields of influenza virus in embryonized chicken eggs, the high specificity for a very limited number of influenza A subtypes, and the inability to use vaccines in individuals (mainly children) with egg allergies (Lipatov et al., 2004).

A number of attempts have been made to develop vaccines based on recombinant proteins derived from HA or NA. However all of these vaccines have limitations with respect to efficiency and efficacy of treatment. Wei et al. (J Virol. 2008) describe vaccination of mice with recombinant H5N1 HA proteins. Plasmids for expression of the recombinant H5N1 HA proteins contain nucleic acid sequences encoding a trimerization domain and a His-tag, which are both removed after purification of the proteins. To be effective the mice had to be immunized more than once (week 0 and 3) with relatively high amounts (20 µg) of protein. In Song et al. (PLoS One. 2008) and WO 2007/103322 immunization of mice with two doses of a composition comprising the globular head of HA fused to TLR5 ligand flagellin is described. Additionally, in Song et al. (PLoS One. 2008) rabbits are immunized with two doses of 5 or 15 µg HA fused to flagellin. In Saelens et al. (Eur J Biochem. 1999) three doses are used of a composition comprising recombinant HA expressed in *P. pastoris* and Ribi adjuvant, an oil-in-water emulsion, for immunizing mice, and WO 95/18861 describes the use of three doses of a composition comprising neuraminidase for immunizing mice. Kong et al. (Proc Natl Acad Sci USA. 2006) need three doses to immunize mice with plasmid DNA expressing an influenza virus hemagglutinin, as is also described in WO 2007/100584, WO 2008/112017 and WO 2009/092038.

It is an object of the present invention to provide improved immunogenic compositions for eliciting a protective immune response against an influenza virus. The invention therefore provides an immunogenic composition comprising at least one recombinant multimeric ectodomain of an influenza virus protein or part thereof fused to a streptavidin affinity tag, and a pharmaceutically acceptable diluent, wherein said recombinant multimeric ectodomain of an influenza virus protein or part thereof is selected from the group consisting of
- a recombinant trimeric influenza hemagglutinin ectodomain or part thereof and
- a recombinant tetrameric influenza neuraminidase ectodomain or part thereof.

An "immunogenic composition" is herein defined as a composition that is capable of eliciting an immune response when administered to an individual. The elicited immune response can be humoral, cellular or a combination thereof and includes, but is not limited to, the production of antibodies, B cells, and T cells. An immune response as used herein is preferably directed specifically to one or more immunogens of a composition according to the invention.

An immunogenic composition of the present invention can be administered to an individual by any technique known in the art including, but not limited to, intramuscular (IM), intradermal (ID), subcutaneous (SC), intracranial (IC), intraperitoneal (IP), or intravenous (IV) injection, transdermal, oral, intranasal, or rectal administration, and combinations thereof. Furthermore administration of an immunogenic composition according to the invention can be performed individually, such as by injection or oral or nasal administration, or collectively, such as by supplementing drinking water or by spraying onto animals or into the air above animals, for example farm animals. In a preferred embodiment an immunogenic composition according to the invention is used for eliciting an immune response. It is preferred that the immunogenic composition is used as a vaccine.

An "individual" is herein defined as a human or an animal, preferably an animal that can be infected by influenza virus, such as birds, including but not limited to poultry. Individuals include but not limited to chickens, ducks, geese, turkeys, swans, emus, guinea fowls and pheasants, humans, pigs, ferrets, seals, rabbits, cats, dogs and horses. In a preferred embodiment of the invention an individual is a mammal, such as a human, a pig or a horse, or a bird. In one embodiment of the invention said mammal is a human.

A "multimeric ectodomain" is herein defined as an extracellular part of a surface protein which surface protein is composed of at least two (non-covalently bound) subunits. Said surface protein can be any surface protein of an influenza virus. By using a multimeric ectodomain of an influenza virus surface protein or part thereof the correct secondary, tertiary and quaternary structure of said influenza virus surface protein is obtained and/or preserved. An "influenza protein" is herein defined as any protein encoded by the influenza viral genome.

"A pharmaceutically acceptable diluent" as used herein is defined as any solution, substance or combination thereof that has no biologically or otherwise unwanted activity, meaning that it can be administered to an individual together with other components of an immunological composition without causing a substantial adverse reaction.

Throughout the application a recombinant multimeric ectodomain of an influenza protein or part thereof fused to a streptavidin affinity tag is further also referred to as a recombinant multimeric ectodomain or part thereof according to the invention.

As used herein an "influenza virus" encompasses influenza virus type A, influenza virus type B and influenza virus type C and all influenza virus subtypes. "Influenza type" as used herein refers to influenza type A, type B or type C. "Influenza strain" as used herein refers to different influenza A viruses, for example H1N1, H1N2, H1N7, H2N2, H3N2, H3N8, H4N8, H5N1, H5N2, H5N9, H6N2, H6N5, H7N2, H7N3, H7N7, H8N4, H9N2, H10N7, H11N6, H12N5, H13N6. "Hemagglutinin" or "HA" as used herein refers to a hemagglutinin protein of influenza virus and may be of any influenza type, A, B or C, and any serotype, for example H1, H2, H3, H4, H5, H6, H7, H8, H9, H10, H11, H12, H13, H14, H15 or H16, including subtypes belonging to the same serotype but with small difference as a result of for example antigenic drift. "Neuraminidase" or "NA" as used herein refers to a neuraminidase protein of influenza virus and may be of any influenza type, A, B or C, and any serotype, for example, N1, N2, N3, N4, N5, N6, N7, N8 or N9, including subtypes belonging to the same serotype but with small difference as a result of for example antigenic drift. "Part thereof" as used herein is defined as any part of a recombinant multimeric ectodomain of an influenza virus protein that is smaller than an entire multimeric ectodomain of an influenza virus protein, but has a similar immunogenic activity.

For example, a part of a recombinant multimeric HA according to the invention comprises a HA2, or a part of a HA2 comprising a highly conserved epitope proximate to the HA1 subunit of HA (Ekiert et al., 2009), or a HA ectodomain lacking the globular head domain. Preferably said epitope of a HA2, part of a HA2 or HA ectodomain lacking the globular head domain comprises amino acids 1-68 of HA fused to amino acids 293-524 of HA (amino acid numbering according to the numbering of HA of influenza strain X31, EMBL-EBI Accession no. P03438). Because said epitope of HA is highly conserved in different influenza virus types subtypes and strains a wide protection against different influenza virus types, subtypes or strains is provided if said part is used in an immunogenic composition according to the invention.

In a preferred embodiment a use of the immunogenic composition according to the invention for the preparation of a prophylactic agent for eliciting an immune response against an influenza virus is provided. Also provided is an immunogenic composition according to the invention for use as a vaccine.

A "streptavidin affinity tag" or "strep-tag" is herein defined as a tag that binds to streptavidin or a derivative thereof, such as Strep-Tactin, comprising at least one peptide consisting of nine amino acids: Ala-Trp-Arg-His-Pro-Gln-Phe-Gly-Gly (SEQ ID NO: 1), or at least one peptide consisting of eight amino acids (also called strep-tag II): Trp-Ser-His-Pro-Gln-Phe-Glu-Lys (SEQ ID NO: 2), wherein Trp is tryptophan, Ser is serine, His is histidine, Pro is proline, Gln is glutamine, Phe is phenylalanine, Glu is glutamine, Lys is lysine, Ala is alanine, Arg is arginine and Gly is glycine. In a preferred embodiment a streptavidin affinity tag comprises two of more of said peptides, each consisting of eight or nine amino acids, more preferably three of said peptides. In one embodiment a streptavidin affinity tag comprises more than three of said peptides. A strep-tag is, for instance, used for isolation and/or purification purposes. The tag binds to streptavidin or a derivative thereof, for instance immobilized on a column. Elution of a protein fused to a strep-tag from the column can be performed using biotin or a derivative or homologue thereof, such as desthio-biotin. The streptavidin affinity tag can be placed at the C- or N-terminus of the protein of interest. A 'derivative' as used herein is defined as any variant of streptavidin which has the capacity to be bound by a streptavidin affinity tag, and can thus be used to isolate or purify a protein containing a streptavidin affinity tag. A preferred derivative of streptavidin is Strep-Tactin.

Elution of the bound recombinant proteins can be performed under mild conditions by competition with biotin or a suitable derivative. With mild conditions it is meant that purification can be performed under physiological conditions, such as aqueous solutions, room temperature, neutral or slightly basic pH, and normal pressure.

According to the invention a recombinant multimeric ectodomain of an influenza protein or part thereof is selected from the group consisting of a recombinant trimeric influenza hemagglutinin ectodomain or part thereof and a recombinant tetrameric influenza neuraminidase ectodomain or part thereof.

In some embodiments an amino acid sequence of a recombinant multimeric ectodomain or part thereof according to the invention is altered such as by amino acid substitutions, deletions and/or additions that provide proteins functionally equivalent to a recombinant multimeric ectodomain or part thereof according to the invention. Functionally equivalent proteins can be identified by any method known in the art, for example by their ability to induce an immune response against influenza virus in an individual, or in an in vitro assay wherein binding to antibodies is determined. An altered recombinant multimeric ectodomain is provided in an immunogenic composition according to the invention for example because it can be produced at a higher level, compared to a non-altered parent multimeric ectodomain.

Recombinant multimeric ectodomains or parts thereof according to the invention are preferred over monomeric ectodomains or parts thereof of influenza virus proteins because such multimeric ectodomains will result in more effective elicitation of an immune response. Therefore, a lower dosage and a reduced number of administrations of an immunogenic composition comprising such recombinant multimeric ectodomain according to the invention is needed in order to elicit a sufficient immune response, compared to immunogenic compositions comprising a monomeric ectodomain of an influenza virus protein.

An immunogenic composition according to the invention may comprise one recombinant trimeric influenza hemagglutinin ectodomain or part thereof or one recombinant tetrameric influenza neuraminidase ectodomain or part thereof. Such immunogenic composition is particularly suitable for eliciting an immune response in an individual against an influenza virus subtype or strain, or more than one related influenza virus subtypes or strains. In some embodiments however, an immunogenic composition according to the invention comprises a combination of one or more trimeric hemagglutinin ectodomains or parts thereof, and/or one or more tetrameric neuraminidase ectodomains or parts thereof.

Thus for example, 2, 3, 4, 5, 6, 7, 8, 9 or 10 trimeric hemagglutinin ectodomains or parts thereof can be combined, such as H1, H2, H3, H4, H5, H6, H7, H8, H9, H10, H11, H12, H13, H14, H15 and/or H16, or 2, 3, 4, 5, 6, 7, 8 or 9 tetrameric neuraminidase ectodomains or parts thereof can be combined, such as N1, N2, N3, N4, N5, N6, N7, N8 and/or N9. Additionally, an immunogenic composition according to the invention may comprise a combination of one or more trimeric hemagglutinin ectodomains or parts thereof and one or more tetrameric neuraminidase ectodomains or parts thereof of different influenza subtypes. For example, 1, 2, 3, 4, 5, 6, 7, 8, 9 or 10 trimeric hemagglutinin ectodomains or parts thereof and 1, 2, 3, 4, 5, 6, 7, 8 or 9 tetrameric neuraminidase ectodomains or parts thereof can be combined, such as H1, H2, H3, H4, H5, H6, H7, H8, H9, H10, H11, H12, H13, H14, H15, H16, N1, N2, N3, N4, N5, N6, N7, N8 and/or N9. It will be clear to a skilled person that the term "one or more ectodomains" encompasses one or more ectodomains from one or more influenza virus subtypes, such as for example Influenza A virus and Influenza B virus. Thus, an immunogenic composition according to the invention can be a multivalent composition with which an improved protection against an influenza virus infection can be achieved as compared to an immunogenic composition comprising one recombinant trimeric influenza hemagglutinin ectodomain or part thereof or one recombinant tetrameric influenza neuraminidase ectodomain or part thereof. Additionally, or alternatively, with a multivalent composition a wider protection against more than one influenza virus type, or influenza virus subtypes or strains can be achieved. Furthermore, an immunogenic composition according to the invention may be less sensitive to antigenic alterations than traditional inactivated or live attenuated influenza virus vaccines.

Other advantages of immunogenic compositions according to the present invention are for example the possibility to produce the recombinant multimeric ectodomains or parts thereof in large quantities in cell culture, and at relatively low costs since no high-level biocontainment facilities are necessary for production of immunogenic compositions according to the invention.

In contrast to prior attempts to produce vaccines based on recombinant influenza virus proteins, only very low amounts of a recombinant multimeric ectodomain or part thereof of an influenza virus protein or part thereof fused to a streptavidin affinity tag according to the invention are necessary for eliciting an immune response in an individual. As is shown in more detail in the examples, amounts as low as 3.75 µg of recombinant protein in ferrets, 5 µg of recombinant protein in swine and 0.4 µg of recombinant protein in chickens are sufficient to elicit a protective immune response in these respective animals upon challenge with influenza virus.

Immunogenic compositions according to the invention can be administered in a single dose or in multiple doses. However, as opposed to other vaccines based on recombinant influenza proteins, protection against an influenza virus infection is achieved after one administration of an immunogenic composition according to the invention. Thus, immunogenic compositions according to the present invention are more effective than other immunogenic compositions based on recombinant influenza proteins known in the art.

A strep-tag is not likely to influence protein function, structure, folding, secretion, and has a low immunogenicity. In addition, elution of a strep-tag containing protein is achieved under mild conditions. Without being bound by theory, it is believed that a combination of at least one recombinant multimeric ectodomain according to the invention and the use of a streptavidin affinity tag results in the advantages of the present invention over immunogenic composition comprising influenza proteins known in the art, possibly because of the mild conditions that can be employed during the purifications of proteins and, hence, the good conservation of the proteins' antigenicity and the more effective elicitation of an immune response.

In some embodiments of the invention a trimerization domain is used to provide a trimeric recombinant influenza hemagglutinin ectodomain or part thereof. The term "trimerization domain" as used herein is defined as a domain that mediates the formation of a trimer out of three monomeric proteins or parts thereof. Suitable trimerization domains include, but are not limited to, a trimerization sequence from bacteriophage T4 fibritin and an artificial trimerization domain (GCN4-pII) based on the yeast GCN4 coiled coil protein. In a preferred embodiment, trimerization of a recombinant influenza hemagglutinin ectodomain or part thereof is provided by a GNC4-based trimerization domain. Said GCN4-based trimerization domain preferably comprises the amino acid sequence: MKQIEDKIEEIESKQKKIENEIARIKK (SEQ ID NO: 3).

In some embodiments of the invention a tetramerization domain is used to provide a tetrameric recombinant influenza neuraminidase ectodomain or part thereof. The term "tetramerization domain" as used herein is defined as a domain that mediates the formation of a tetramer out of four monomeric proteins or parts thereof. Suitable tetramerization domains include, but are not limited to, the Sendai virus phosphoprotein tetramerization domain and a tetramerization domain (GCN4-pLI) derived by mutation from the yeast GCN4 dimerization domain. In a preferred embodiment, tetramerization of a recombinant influenza neuraminidase ectodomain or part thereof is provided by a GCN4-based tetramerization domain. A GCN4-based tetramerization domain preferably comprises the amino acid sequence: MKQIEDKLEEILSKLYHIENELARIKKLLGE (SEQ ID NO: 4).

In some embodiments an immunogenic composition according to the invention comprises an adjuvant and/or a carrier or other excipient known in the art. "An adjuvant" is defined herein as a substance added to an immunological composition in order to enhance the immune response of an individual to a particular antigen at either the cellular of humoral level. Suitable adjuvants include, but are not limited to aluminium and calcium salts such as aluminium phosphate, aluminium hydroxide, aluminium potassium sulphate (alum) and calcium phosphate, organic adjuvants such as Squalene, oil-based adjuvants such as complete and incomplete Freund's adjuvant, RIBI Adjuvant System® (RAS®), Stimune (Specol)®, TiterMax®, Montanides, MF-59, AS03, QS21, Adjuvant 65, saponin, MDP, and Syntex Adjuvant Formulation (SAF)®, monophosphoryl lipid A, Gerbu® Adjuvant, immune-stimulating complexes (ISCOMs), cytokines such as Interferon-gamma, immunostimulatory nucleic acid sequences such as CpG oligonucleotides, liposomes, virus-like particles, surfactants such as hexadecylamine, polyanions such as pyran and dextran sulfate. A preferred adjuvant is Stimune (Specol). Further preferred adjuvants for use in humans are aluminium phosphate or hydroxide, calcium phosphate, ISCOMs, AS03 or MF59.

A preferred adjuvant is ISCOMs. ISCOM technology has several advantages over other adjuvants. ISCOMs stimulate both humoral and cell-mediated immune responses. ISCOMs is a highly efficient adjuvant, enabling further reduction of the amounts of a recombinant multimeric ectodomain or part thereof according to the invention. A preferred ISCOMs is ISCOM Matrix-M. Without being bound to theory it is possible that the use of ISCOMs in a method or immunogenic composition according to the present invention may even enhance the advantages of the present invention over immunogenic composition comprising influenza proteins known in the art, such as a lower dosage and a reduced number of administrations of an immunogenic composition according to the invention.

As used herein "a carrier" or "a scaffold" is defined as a molecule such as a peptide, polypeptide, protein, protein complex, or chemical compound that is able to associate with any component of immunogenic compositions as defined herein for the purpose of transport of said component, presentation to the immune system, and/or enhancing the immunogenicity of said immunogenic composition. Suitable carriers or scaffolds include but are not limited to a biological scaffold such as a virus, a bacteria, a virus-like particle and a bacterial remnant such as a bacterial wall skeleton, a chemical scaffold such as an array of chemically linked streptavidin units or derivatives thereof, a biochemical multivalent linker, a diphtheria or tetanus toxoid, Keyhole limpet hemocyanin (KLH), serum albumin (such as BSA), hemocyanin, albumine, non-toxic mutant diphtheria toxin CRM197, outer membrane protein complex (OMPC) from Neisseria meningitidis, the B subunit of heat-labile Escherichia coli, a virus-like particle assembled from recombinant coat protein of bacteriophage Qb and cellulose beads.

The present invention further provides a vector comprising an expression cassette comprising a nucleic acid sequence encoding a signal sequence, a nucleic acid sequence encoding a recombinant ectodomain of an influenza protein or part thereof, a nucleic acid sequence encoding a trimerization or tetramerization sequence, and a nucleic acid sequence encoding a streptavidin affinity tag.

A vector according to the invention is particularly suitable for expression and production of a recombinant multimeric ectodomain or part thereof according to the invention. Therefore, in a preferred embodiment, a use of a vector according to the invention for the preparation of an immunogenic composition according to the invention for eliciting an immune response against an influenza virus is provided. Also provided is a vector according to the invention for use as a vaccine.

The term "vector" as used herein is defined as a nucleic acid molecule, such as a plasmid, bacteriophage or animal virus, capable of introducing a heterologous nucleic acid sequence into a host cell. A vector according to the invention allows the expression or production of a protein or part thereof encoded by the heterologous nucleic acid sequence in a host cell. Vectors suitable in a method according to the invention include, but are not limited to, pCD5 (Pouyani and Seed, 1995) and pCAGGS (Niwa et al., 1991). A "host cell" is a cell which has been transformed, or is capable of transformation, by a nucleic acid molecule such as a vector. The term "transformation" is herein defined as the introduction of a foreign nucleic acid into a recipient cell. Transformation of a recipient cell can result in transient expression of a recombinant protein by said cell, meaning that the recombinant protein is only expressed for a defined period of time. Alternatively, transformation of a recipient cell can result in stable expression, meaning that the nucleic acid is introduced into the genome of the cell and thus passed on to next generations of cells. Additionally, inducible expression of a recombinant protein can be achieved. An inducible expression system requires the presence or absence of a molecule that allows for expression of a nucleic acid sequence of interest. Examples of inducible expression systems include, but are not limited to, Tet-On and Tet-Off expression systems, hormone inducible gene expression system such as for instance an ecdysone inducible gene expression system, an arabinose-inducible gene expression system, and a Drosophila inducible expression system using a pMT/BiP vector (Invitrogen) which comprises an inducible metallothioneine promoter.

A "signal sequence" is herein defined as a signal peptide that directs transport of a protein or part thereof to the endoplasmic reticulum of a eukaryotic cell - the entry site of the secretory pathway. A signal sequence can be located N-terminal or C-terminal of the protein or part thereof. Signal peptides can be cleaved off after insertion of the protein into the endoplasmic reticulum. For example, in one embodiment a pMT/BiP vector for expression of recombinant protein in Drosophila cells comprises a BiP signal sequence. In another embodiment, a pCD5 vector for expression of recombinant protein in mammalian cells comprises the signal sequence: MPMGSLQPLATLYLLGMLVA (SEQ ID NO:5).

An expression cassette preferably comprises a promoter/enhancer which allows for initiation of transcription of nucleic acid sequences further contained within the expression cassette. Examples of promoters include, but are not limited to, RAmy3D and CaMV promoters for expression in plant cells, polyhedrin, p10, IE-0, PCNA, OplE2, OplE1, and Actin 5c promoters for expression in insect cells, and beta-actin promoter, immunoglobin promoter, 5S RNA promoter, or virus derived promoters such as cytomegalovirus (CMV), Rous sarcoma virus (RSV), and Simian virus 40 (SV40) promoters for expression in mammalian cells. A preferred vector is provided by the pCD5 expression vector for expression of recombinant protein in mammalian cells comprising a CMV promoter. A further preferred vector is provided by a pCAGGS expression vector for expression of recombinant protein in mammalian cells comprising a CMV enhancer / chicken beta-actin (CAG) promoter.

An expression cassette according to the invention which is intended for the expression of a trimeric influenza hemagglutinin ectodomain or part thereof preferably comprises from 5' to 3': a nucleic acid sequence encoding a signal sequence; a nucleic acid sequence encoding a recombinant ectodomain of influenza hemagglutinin or part thereof; a nucleic acid sequence encoding a trimerization sequence, preferably a GCN4-based trimerization sequence, and a nucleic acid sequence encoding a streptavidin affinity tag. A vector according to the invention which is intended for the expression of a tetrameric influenza neuraminidase ectodomain or part thereof preferably comprises from 5' to 3': a nucleic acid sequence encoding a signal sequence; a nucleic acid sequence encoding a streptavidin affinity tag; a nucleic acid sequence encoding a tetramerization sequence, preferably a GCN4-based tetramerization sequence, and; a nucleic acid sequence encoding a recombinant ectodomain of influenza hemagglutinin or part thereof.

A recombinant multimeric ectodomain or part thereof according to the invention is for instance expressed in a prokaryotic cell, or in a eukaryotic cell, such as a plant cell, a yeast cell, a mammalian cell or an insect cell. Additionally a recombinant multimeric ectodomain or part thereof according to the invention can be expressed in plants. Prokaryotic and eukaryotic cells are well known in the art. A prokaryotic cell is for instance *E. coli.* Examples of plants and/or plant cells include, but are not limited to, corn (cells), rice (cells), duckweed(cells), tobacco (cells, such as BY-2 or NT-1 cells), and potato(cells). Examples of yeast cells are *Saccharomyces* and *Pichia.* Examples of insect cells include, but are not limited to, *Spodoptera frugiperda* cells, such as Tn5, SF-9 and SF-21 cells, and Drosophila cells, such as Drosophila Schneider 2 (S2) cells. Examples of mammalian cells that are suitable for expressing a recombinant multimeric ectodomain or part thereof according to the invention include, but are not limited to, African Green Monkey kidney (Vero) cells, baby hamster kidney (such as BHK-21) cells, Human retina cells (for example PerC6 cells), human embryonic kidney cells (such as HEK293 cells), Madin Darby Canine kidney (MDCK) cells, Chicken embryo fibroblasts (CEF), Chicken embryo kidney cells (CEK cells), blastoderm-derived embryonic stem cells (e.g. EB14), mouse embryonic fibroblasts (such as 3T3 cells), Chinese hamster ovary (CHO) cells, mouse myelomas (such as NS0 and SP2/0), quail muscle cells (QM5), and derivatives of these cell types. In a preferred embodiment mammalian HEK293T cells or S2 insect cells are used for production of a recombinant multimeric ectodomain or part thereof according to the invention.

Further provided by the invention is a method for the preparation of an immunogenic composition according to the invention, comprising providing a vector according to the invention, transforming a host cell with said vector, culturing said host cell in a culture medium, thereby allowing expression of the recombinant multimeric ectodomain of an influenza protein or part thereof fused to a streptavidin affinity tag, and isolating said recombinant multimeric ectodomain of an influenza protein or part thereof fused to a streptavidin affinity tag.

When using a method according to the invention for expression of a recombinant multimeric ectodomain or part thereof according to the invention in a host cell said multimeric ectodomain or part thereof is preferably secreted into the culture medium and can be isolated from said culture medium. Isolation of said multimeric ectodomain or part thereof is for instance performed by centrifuging or filtrating the culture medium to remove cells and cell remnants, and purification using any method known in the art, for instance gel electrophoresis or chromatography methods, such as affinity chromatography or high performance liquid chromatography. In a preferred embodiment a recombinant multimeric ectodomain or part thereof according to the invention is purified using the fused streptavidin affinity tag, for instance using purification columns with streptavidin or Strep-Tactin Sepharose (IBA Germany) and an elution buffer for displacing the Strep-tag protein from the column comprising biotin or a derivative or homologue thereof.

In some embodiments a vector according to the invention is derived from an animal virus such as, but not limited to, vaccinia virus (including attenuated derivatives such as the Modified Vaccinia virus Ankara, MVA), Newcastle Disease virus (NDV), adenovirus or retrovirus. It is preferred that an animal virus that used as a vector according to the invention is attenuated. Any of these vectors is particularly suitable for expression of a recombinant multimeric ectodomain or part thereof according to the invention in a host in which an immunogenic response is intended to be elicited. An advantage of the use of an animal virus such as NDV is that it can be efficiently grown in embryonated chicken eggs at low costs and in sufficiently high amounts. In a preferred embodiment, after propagation of the animal virus in embryonated chicken eggs isolated virus is subsequently used to infect eukaryotic cells. Infected cells are then used for production of a recombinant multimeric ectodomain or part thereof according to the invention. Examples of cells that are suitable for expressing a recombinant multimeric ectodomain or part thereof according to the invention are mammalian cells as described above. In another preferred embodiment, after for example propagation of the animal virus in embryonated chicken eggs, the isolated virus is used to directly infect cells of a host in which an immunogenic response is to be elicited. One advantage of infecting a host in which an immunogenic response is to be raised with a vector according to the invention is that administration is very efficiently carried out, for instance by spraying onto animals or into the air above animals. The invention therefore further comprises an immunogenic composition comprising the vector according to the invention, and a pharmaceutically acceptable diluent.

A vector according to the invention for use in an immunogenic composition according to the invention preferably comprises an expression cassette comprising a promoter that is suitable for initiation of transcription in cells of the intended host. Examples of suitable promoters for expression of at least one recombinant multimeric ectodomain or part thereof according to the invention in avian hosts include, but are not limited to, avian beta-actin promoter or a chicken RNA pol I promoter. Examples of suitable promoters for expression of at least one recombinant multimeric ectodomain or part thereof according to the invention in mammalian hosts include, but are not limited to, beta-actin promoter, immunoglobin promoter, 5S RNA promoter, or virus derived promoters such as cytomegalovirus (CMV), Rous sarcoma virus (RSV) and Simian virus 40 (SV40) promoters for mammalian hosts.

In some embodiments a nucleic acid sequence encoding a recombinant ectodomain or part thereof according to the invention is altered such as by nucleic acid substitutions, deletions and/or additions that enable expression of proteins functionally equivalent to a recombinant multimeric ectodomain or part thereof according to the invention. Functionally equivalent proteins can be identified by any method known in the art, for example by their ability to induce an immune response against influenza virus in an individual, or in an in vitro assay wherein binding to antibodies is determined.

In some embodiments a nucleic acid sequence encoding a trimeric influenza hemagglutinin ectodomain or part hereof, and/or a nucleic acid sequence encoding a tetrameric influenza neuraminidase or part thereof is inserted into a vector. In another embodiment nucleic acid sequences encoding more than one trimeric influenza hemagglutinin ectodomain or part hereof, and/or tetrameric influenza neuraminidase or part thereof or combinations thereof are inserted into a vector. In yet another embodiment an immunogenic composition according to the invention comprises separate vectors in each of which a nucleic acid sequence encoding a trimeric influenza hemagglutinin ectodomain or part hereof, and/or a tetrameric influenza neuraminidase or part thereof is inserted.

Thus, for example 2, 3, 4, 5, 6, 7, 8, 9 or 10 nucleic acid sequences encoding a trimeric hemagglutinin ectodomain or part thereof can be combined in one vector, such as nucleic acid sequences encoding H1, H2, H3, H4, H5, H6, H7, H8, H9, H10, H11, H12, H13, H14, H15 and/or H16. Alternatively 2, 3, 4, 5, 6, 7, 8 or 9 nucleic acid sequences encoding a tetrameric neuraminidase ectodomain or part thereof can be combined in one vector, such as nucleic acid sequences encoding N1, N2, N3, N4, N5, N6, N7, N8 and/or N9. Additionally, an immunogenic composition according to the invention may comprise a combination of one or more nucleic acid sequences encoding a trimeric hemagglutinin ectodomain or part thereof and one or more nucleic acid sequences encoding a tetrameric neuraminidase ectodomain or part thereof of different influenza subtypes. For example, 1, 2, 3, 4, 5, 6, 7, 8, 9 or 10 nucleic acid sequences encoding trimeric hemagglutinin ectodomains or parts thereof and 1, 2, 3, 4, 5, 6, 7, 8 or 9 nucleic acid sequences encoding tetrameric neuraminidase ectodomains or parts thereof can be combined, such as H1, H2, H3, H4, H5, H6, H7, H8, H9, H10, H11, H12, H13, H14, H15, H16, N1, N2, N3, N4, N5, N6, N7, N8 and/or N9. It will be clear to a skilled person that the term "one or more ectodomains" encompasses one or more ectodomains from one or more influenza virus subtypes, such as for example Influenza A virus and Influenza B virus. This way, an immunogenic composition according to the invention may comprise a multivalent composition with which a wider protection against more than one influenza virus type, or influenza virus subtypes or strains is achieved. Furthermore, such an immunogenic composition may be less sensitive to antigenic alterations than traditional inactivated or live attenuated influenza virus vaccines.

Further provided by the invention is a method for eliciting an immune response against an influenza virus in an individual comprising administering to said individual an immunogenic composition according to the invention.

The invention is further explained in the following examples. These examples do not limit the scope of the invention, but merely serve to clarify the invention.

### Figure legends

**Figure 1****.** Schematic representation of an influenza virus.

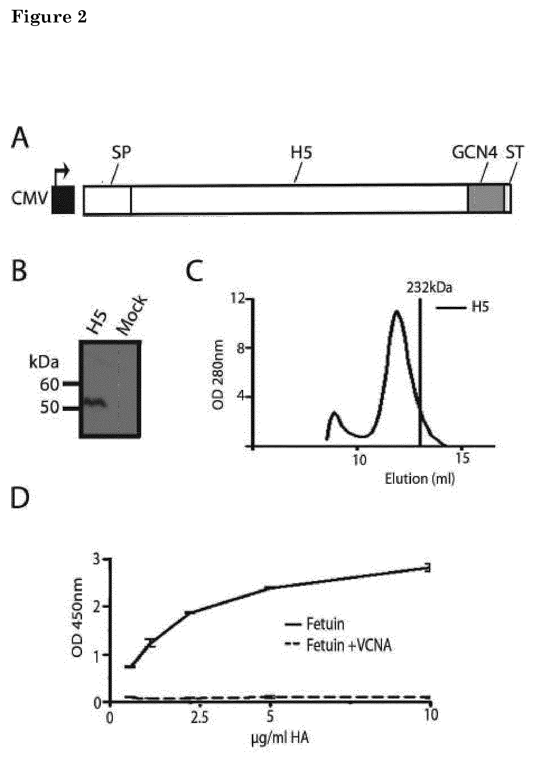

**Figure 2****.** Expression, purification and biological activity of recombinant, soluble trimeric H5 proteins. (A) Schematic representation of the H5 expression cassettes used. The H5 ectodomain encoding sequence (H5) was cloned in frame with DNA sequences coding for a signal peptide (SP), the GCN4 isoleucine zipper trimerization motif (GCN4) and the Strep-tagII (ST) under the control of a CMV promoter. (B) H5 expression and secretion into the culture media was analyzed by SDS-PAGE followed by western blotting. The recombinant protein was detected using a mouse anti-Strep-tag antibody. (C) Analysis of purified recombinant H5 proteins by gel filtration. The elution profiles of the H5 protein using a Superdex200GL 10-300 column are shown. The elution of a 232kDa catalase control is indicated by the dotted line. (D) Recombinant soluble H5 trimers were complexed with a HRP-conjugated, mouse antibody directed against the Strep-tag prior to their application in a fetuin binding assay. HA binding was also assessed after treatment of fetuin with *Vibrio Cholera* derived neuraminidase (fetuin + VCNA).

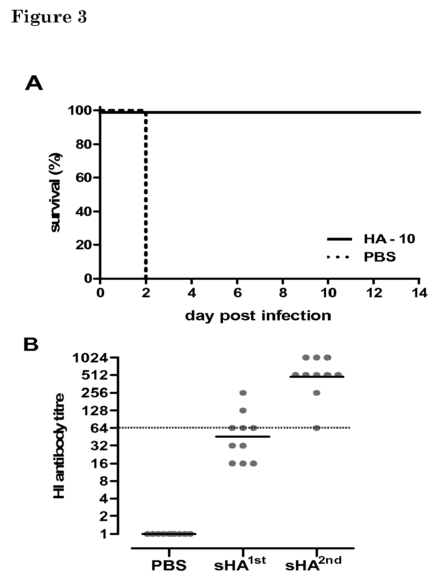

**Figure 3****.** Soluble hemagglutinin (sHA) vaccination in chickens. One group of 10 SPF chickens was immunized two times with 10 ug sHA (on d0 and d21). As a challenge control, one group of chickens was mock-treated (PBS). Three weeks after the 2^{nd} vaccination, all birds were challenged with 10⁵ TCID₅₀ of H5N1 A/Vietnam/1203/04 and monitored daily for clinical signs until day 14 p.i. A) Kaplan-Meier survival curve, indicating percentage mortality on each day for each group. B) Blood samples were collected 3 weeks after the first immunization (d21), 3 weeks after the second vaccination (d42) and 2 weeks post challenge (d56). Graphed are HI antibody titers in serum for each bird. Bars represent geometric means for each group. The dotted line indicates the lowest antibody level correlated with protection in this experiment.

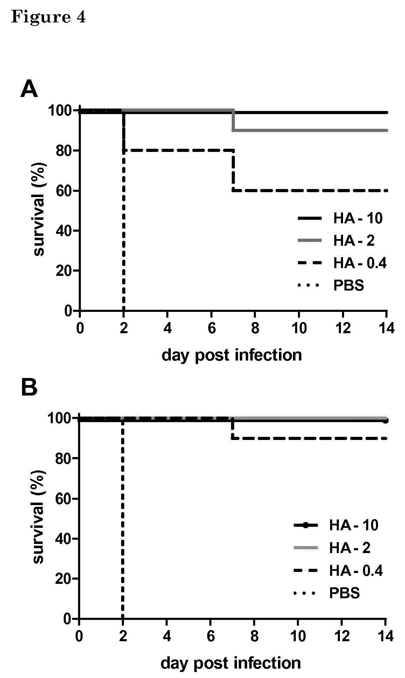

Figure 4. sHA vaccination in chickens. Seven groups of 10 WLH chickens were immunized i.m. with 10, 2 or 0.4 µg sHA either once or twice with a 3 weeks interval. As a challenge control, one group was mock-treated (PBS). Four weeks after the vaccination, all chickens were challenged with 10⁵ TCID₅₀ of A/Vietnam/1203/04 and then monitored daily for clinical signs during 14 days. Kaplan-Meier survival curve indicating percentage mortality on each day for each group that was vaccinated once (A) or twice (B).

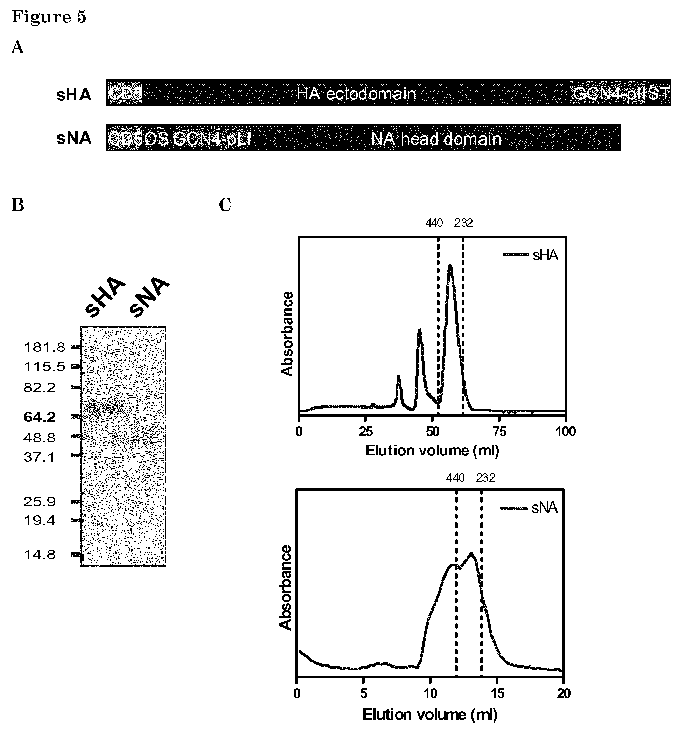

**Figure 5****.** Expression and analyses of purified soluble, multimeric HA (sHA) and NA (sNA) proteins of 2009 A(H1N1) influenza virus. A) Schematic representation of the recombinantly expressed sHA and sNA subunits. sHA: the HA ectodomain (a.a. 17-522) is expressed with an N-terminal CD5 signal sequence and a C-terminal trimeric (GCN4-pII) GCN4 domain and *Strep*-Tag (ST), respectively. sNA: the NA head domain (a.a. 75-469) is expressed with an N-terminal CD5 signal sequence, a OneSTrEP (OS) and a tetrameric (GCN4-pLI) GCN4 domain. (B) Coomassie blue-stained reducing SDS-PAGE of affinity-purified sHA and sNA proteins. (C) Gel filtration chromatography of purified sHA and sNA proteins on Superdex 200 16/600 and Superdex 200 10/300 column, respectively. Dashed lines indicate migration of calibration standards (ferritin, 440 kDa; catalase, 232 kDa). The apparent molecular mass of the main peaks is 288 kDa for sHA and 291 kDa for sNA correlating with a trimeric and tetrameric oligomeric state of sHA and sNA, respectively.

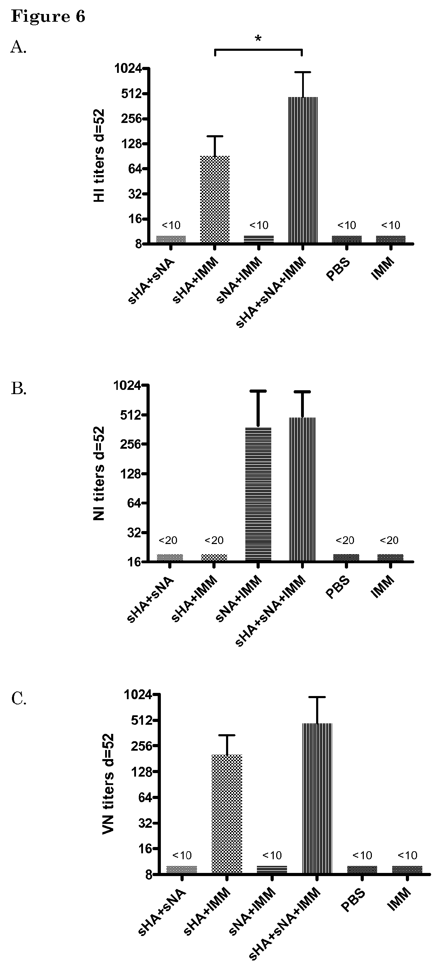

**Figure 6****.** Induction of antibodies to 2009 A(H1N1) influenza virus after vaccination in ferrets. (A) Hemagglutination inhibition titers (HI), (B) neuraminidase inhibition titers (NI) and (C) virus neutralizing titers (VN) of sera of ferrets that received an immunization on day 0 and day 21 with: 3.75 µg HA+3.75 µg NA, 3.75 µg HA with adjuvant (Iscoms Matrix M; [IMM]), 3.75 µg NA+IMM, 3.75 µg HA+3.75 µg NA+IMM, PBS or IMM, as indicated. Geometric mean titers are displayed; error bars indicate SD. Statistical significant differences are indicated: *, P < 0.05.

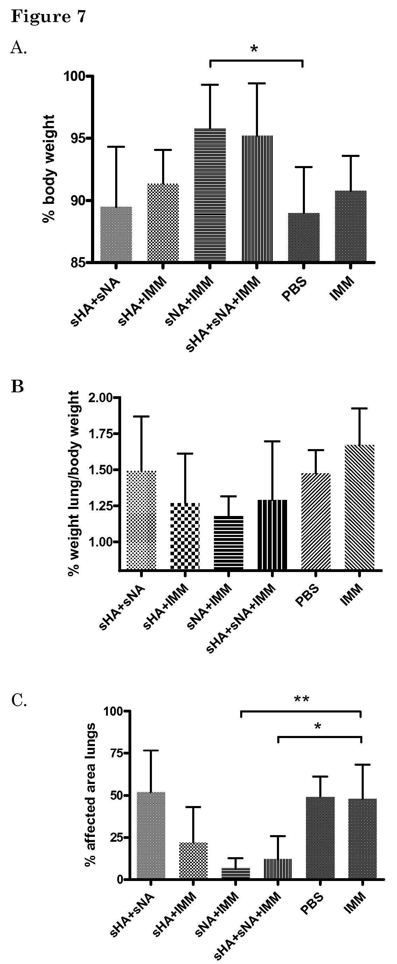

**Figure 7****.** Animal weight and lung pathology of ferrets inoculated with 2009 A(H1N1) influenza virus. Six groups of six ferrets each were immunized twice with: 3.75 µg HA+3.75 µg NA, 3.75 µg HA with adjuvant (Iscoms Matrix M; [IMM]), 3.75 µg NA+ IMM, 3.75 µg HA+3.75 µg NA+ IMM, PBS or IMM as indicated. Animals were inoculated intranasally on day 56 with 10⁶ TCID₅₀ of virus. (A) Weight loss of inoculated animals is indicated as a percentage of body weight before infection (100%). (B) The lung weight as a percentage of body weight, as an indicator for lung consolidation. (C) Lungs were observed macroscopically and scored for lung area percentage displaying necrotic lesions. Geometric mean titers are displayed; error bars indicate SD. Statistical significant differences are indicated: *, P < 0.05; **, P < 0.01.

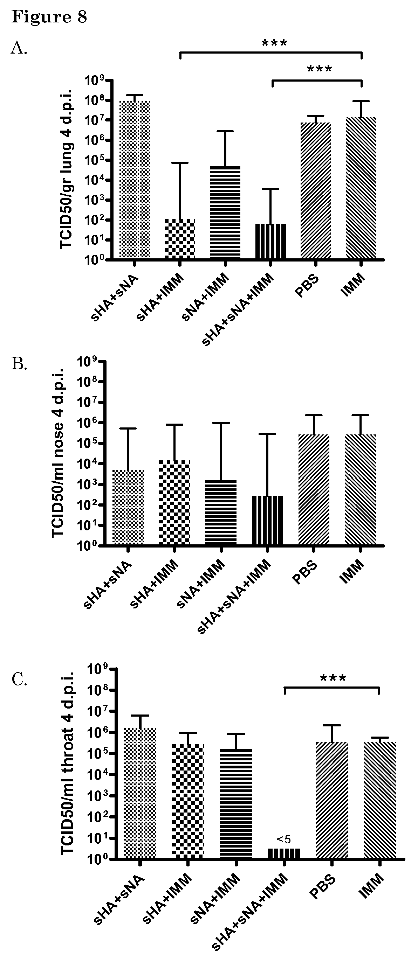

**Figure 8****.** Shedding of 2009 A(H1N1) influenza virus in the lungs, nose and throat of inoculated ferrets. (A) Lung virus titers on day 4 post infection with 2009 A(H1N1) influenza virus in ferrets immunized with 3.75 µg HA+3.75 µg NA, 3.75 µg HA with adjuvant (Iscoms Matrix M; [IMM]), 3.75 µg NA+ IMM, 3.75 µg HA+3.75 µg NA+ IMM PBS or IMM as indicated. (B and C) Virus shedding from the nose and throat of the inoculated animals. Swabs were collected at day 4 after inoculation. Virus titers were determined by means of end-point titration in MDCK cells. Geometric mean titers are given; error bars indicate standard deviation. Statistical significant differences are indicated: ***, P < 0.001.

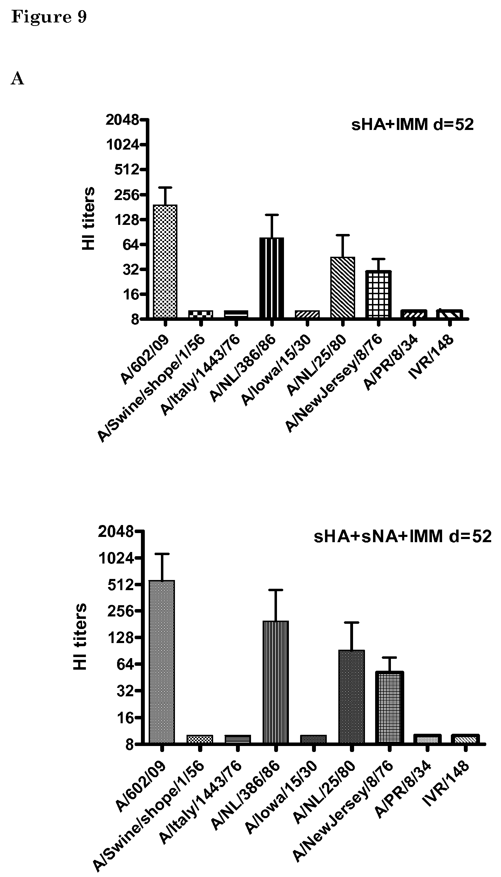

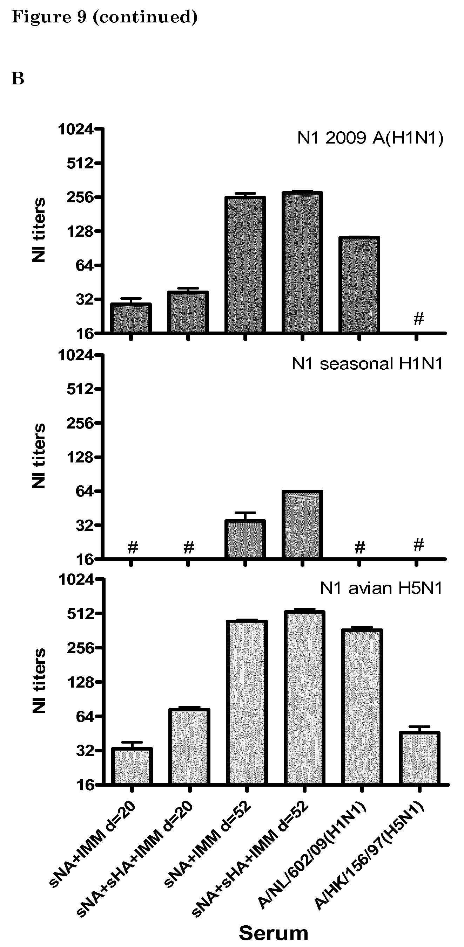

**Figure 9****.** Induction of cross-neutralizing antibodies after vaccination with sHA and sNA of 2009 A(H1N1) influenza virus in ferrets. (A) Sera of ferrets that received a double immunization of sHA or sHA+sNA with adjuvant (Iscoms Matrix M; [IMM]) were tested in a hemagglutinin inhibition (HI) assay for HI antibodies towards different influenza virus isolates including A/Swine/shope/1/56, A/Italy/1443/76, A/NL/386/86, A/Iowa/15/30, A/NL/25/80, A/NewYersey/8/76, A/PR/8/34 and IVR/148 influenza H1N1 viruses. Geometric mean titers are displayed; error bars indicate SD. (B) Sera of ferrets that received a single or double immunization of sNA or sHA+sNA with adjuvant were pooled and tested in a neuraminidase inhibition (NI) assay for NI antibodies against NA of the A/Kentucky/UR06-0258/2007(H1N1) and A/turkey/Turkey/1/2005(H5N1) influenza virus. The NA of A/California/04/2009(H1N1) was taken along as a positive control. Positive control sera specific for A/NL/602/09(H1N1) or A/turkey/Turkey/1/2005(H5N1) influenza virus were obtained from a ferret infected with these viruses. Average titers of two replicates are displayed; error bars indicate SD.

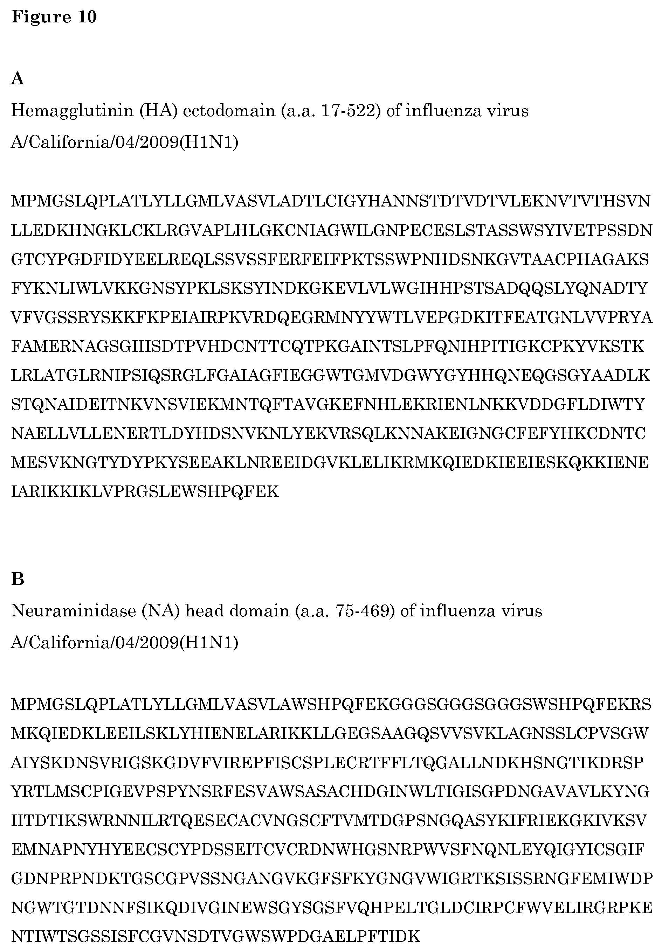

**Figure 10****.** A. Hemagglutinin (HA) ectodomain (a.a. 17-522) of influenza virus A/California/04/2009(H1N1) (SEQ ID NO: 18). B. Neuraminidase (NA) head domain (a.a. 75-469) of influenza virus A/California/04/2009(H1N1) (SEQ ID NO: 19).

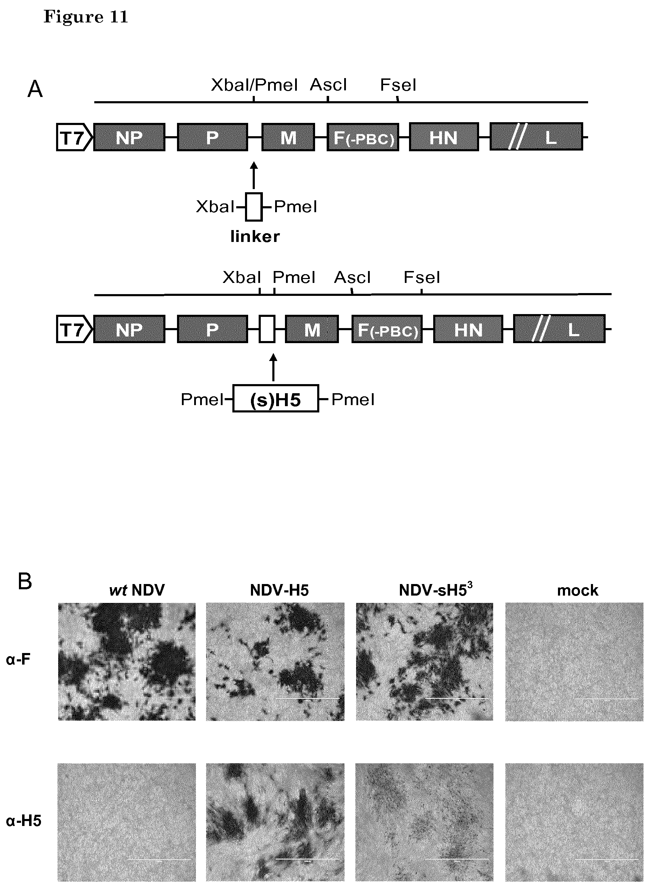

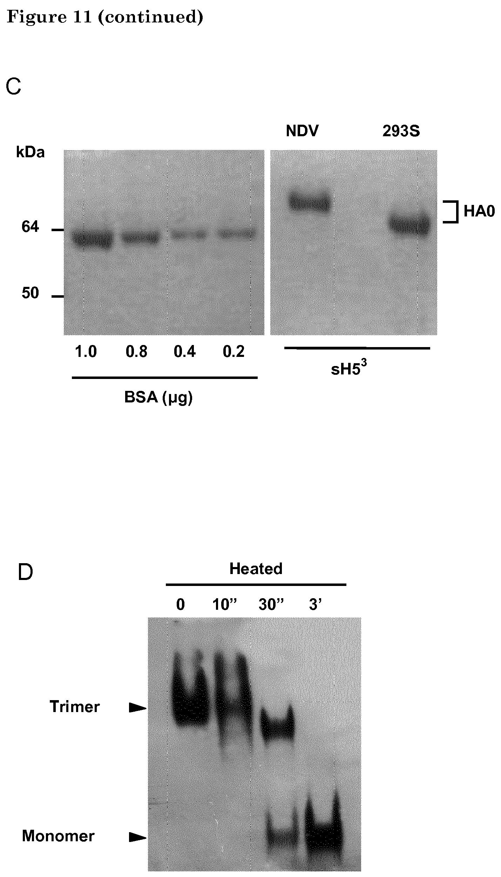

**Figure 11****.** Generation of rNDV-HA vector virus and expression of HA and sH5³. A) Schematic representation of the pFL-HertsΔPBC construct encoding the influenza HA protein. XbaI and PmeI restriction sites were introduced by site directed mutagenesis to enable insertion of a linker containing NDV end-start transcription signals. The genes encoding the unmodified H5 HA gene or the soluble sH5³ protein, the latter consisting of the H5 HA ectodomain fused to the trimerisation sequence GCN4, were each inserted downstream of the linker sequence. B) Expression of HA in monolayers of QM5 cells. Cells were mock-infected or infected with *wt* NDV Herts (indicated as *wt* NDV), rNDV-H5 HA or rNDV-sH5³ (indicated as NDV-H5 and NDV-sH5³, respectively) and stained for HA and F protein using specific antibodies. C) Production and secretion of sH5³ by rNDV-sH5³-infected cells. QM5 cells were infected with rNDV-sH5³ in the absence of trypsin at an MOI of 3 and the supernatant was harvested at 2 days p.i. sH5³ protein was purified from the supernatant using Strep-tactin sepharose beads. The purification product was subjected to SDS-PAGE electrophoresis and proteins were stained with Blue stain reagent. The sH5³ protein expressed in HEK 293S cells and a series of BSA dilutions were run in parallel. D) Blue native PAGE analysis of sH5³ expression product recovered from rNDV-sH5³-infected QM5 cells. The position in the gel of the trimeric and monomeric form of the HA protein observed after heating of the sample prior to electrophoresis is indicated.

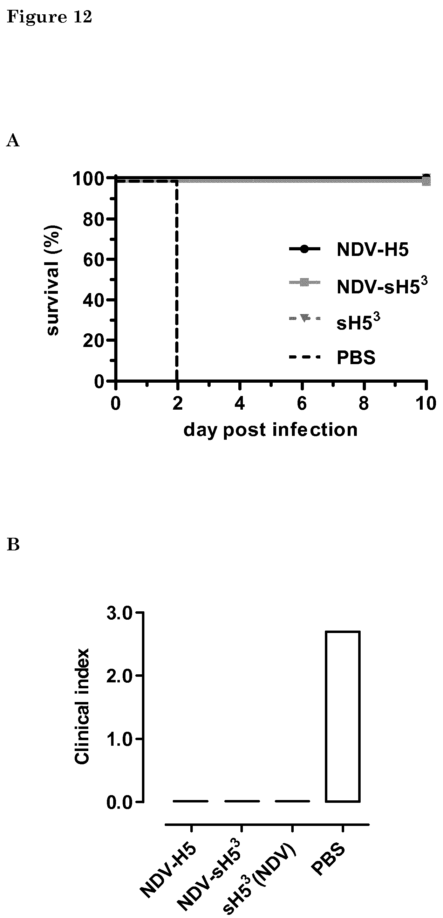

**Figure 12****.** Protection after NDV-H5³ vaccination of chickens. A. Kaplan-Meier survival curves indicating survival (%) of chickens vaccinated intramuscularly (i.m.). B. Clinical index calculated on basis of clinical signs (as described in materials and methods) observed after challenge.
**Figure** 1**3****.** Serological response after NDV-H5³ vaccination of chickens. Hemagglutination inhibition (HI) antibody titers against influenza (A) and NDV Herts (B) measured in serum samples collected on day 21 post vaccination by i.m. route. Titers are expressed as the reciprocal of the highest serum dilution showing HI. The dotted line indicates the lowest limit of detection. Titers below the limit of detection were assigned a value of 2 or 4. Each circle represents one HI measurement per bird for detection of HI antibodies against avian influenza (AI) and two measurements per bird in the case of NDV HI antibody detection. Horizontal lines indicate geometric mean titres per group.

### Examples

### Example 1

### Materials and Methods

### Genes and expression vectors

Based on H3 numbering, a cDNA clone encoding amino acid residues corresponding to residues 18 to 523 of the HA from A/Viet Nam/1203/2004 (H5N1) (Genbank accession no. ABW90137.1) was synthesized using human-preferred codons by GenScript USA Inc. The HA ectodomain encoding cDNA was cloned into the pCD5 expression vector for efficient expression in mammalian cells (Zeng et al., 2008). The pCD5 vector had been modified such that the HA-encoding cDNA was cloned in frame with DNA sequences coding for a signal sequence, a GCN4 isoleucine zipper trimerization motif: ELIKRMKQIEDKIEEIESKQKKIENEIARIKKIKLVPRGSLE (SEQ ID NO: 6) (Pancera et al., 2005), and the Strep-tagII (WSHPQFEK; SEQ ID NO: 2; IBA, Germany).

**Protein expression and purification.** pCD5 expression vector containing the HA ectodomain-encoding sequence was transfected into HEK293S GnTI(-) cells (Reeves et al., 1999) using polyethyleneimine (PEI) in a 1:5 ratio (µg PEI: µg DNA). At 6 h post transfection, the transfection mixture was replaced by 293 SFM II expression medium (Invitrogen), supplemented with sodium bicarbonate (3.7 g/liter), glucose (2.0 g/liter), Primatone RL-UF (3.0 g/liter, Kerry Bio-Science, Zwijndrecht, The Netherlands), penicillin (100 units/ml), Streptomycin (100 µg/ml), glutaMAX (Gibco), and 1,5% DMSO. Tissue culture supernatants were harvested 5-6 days post transfection. HA protein expression and secretion was confirmed by sodium dodecylsulfate (SDS)-polyacrylamide gel electrophoresis (PAGE) followed by western blotting using a mouse anti-Strep-tag antibody (IBA, Germany). Secreted HA proteins were purified using Strep-tactin sepharose beads according to the manufacturer's instructions (IBA, Germany). The concentration of purified protein was determined by using a Nanodrop 1000 spectrophotometer (Isogen Life Sciences) according the manufacturer's instructions.

**Biological characterization of recombinant HA.** Oligomerization status of the HA protein was determined by analyzing the elution profile using a Superdex200GL 10-300 column (U-Protein Express). Functionality of the soluble HA trimer (sHA³) was assessed using a fetuin solid phase assays (Lambre et al., 1991). 1µg/ml fetuin per well was used to coat 96-well nunc maxisorp plates. When indicated in the figure legend fetuin was treated with *Vibrio Cholera* derived neuraminidase (VCNA) followed by extensive washing steps. sHA³ was pre-complexed with horseradish peroxidase (HRP)-linked anti-Strep-tag antibody (2:1 molar ratio) for 30 min at 0 °C prior to incubation of limiting dilutions on the fetuin-coated plates (60 min, room temperature [RT]). HA-binding was subsequently detected using tetramethylbenzidine substrate (BioFX) in an ELISA reader (EL-808 [BioTEK]), reading the OD at 450nm.

### Vaccination-challenge experiments in chickens

SPF White Leghorn chickens (Lohmann) of 6 weeks old were used. The birds were housed in a climatized room under Bsl-3 conditions. In the first experiment, one group of 10 chickens was immunized twice (on day 0 and 21) by i.m. injection (0.5 mL) of 10 µg sHA³ adjuvanted with Stimune (ID -Lelystad, Lelystad, The Netherlands). As a challenge control, a group of 10 chickens received an equal volume of PBS in Stimune (mock vaccinated). Three weeks after the vaccination, the birds were challenged by infection with 10⁵ TCID₅₀ of A/Vietnam/1194/04 virus (0.1 ml intranasally, i.n. and 0.1 ml intratracheally, i.t., UniProt Taxonomy ID: 284217) and monitored over a period of 14 days for signs of disease. Blood samples were collected at 3 weeks after each immunization (on d21 and d42) and on day 14 post infection (p.i.). Trachea and cloaca swabs were taken from each chicken on day 2, 4 and 7 p.i.

In the next experiment, a total of 70 one-day-old layer hens (White Leghorn) purchased from a local breeder was used. The chickens were vaccinated against NDV (Newcastle Disease virus) and IBV (Infectious Bronchitis virus) at the age of one day according to the farm's routine. At the age of 6 weeks, the birds were transported to the Biosafety level (Bsl-) 3 facility and allocated to 7 experimental groups of 10 birds each. Six groups were immunized either by a single intramuscular injection of 10, 2 or 0.4 µg sHA³ antigen on day 21 or twice (on day 0 and 21) with the same doses. As a challenge control, one group of 10 birds was mock-vaccinated (on day 0 and 21) with PBS in Stimune. Four weeks after vaccination (day 49), chickens were challenged as above and observed daily for clinical signs during 14 days. Blood samples were taken before vaccination, after the first (day 21) and second (day 49) vaccination and on day 14 p.i. Trachea and cloaca swab samples were taken on the same days as described above.

### Virus detection assay

Trachea and cloaca swabs were stored in cold tryptose broth medium. The medium was clarified by low-speed centrifugation and the supernatant was harvested, aliquoted and stored at -70°C. Upon freeze-thawing, trachea and cloaca swabs sampled from the same bird on the same day were pooled, RNA was extracted and the presence of viral RNA was assayed using quantitative RT-PCR assay (Taq Man™ PCR) directed against the M1 gene, encoding an influenza virus matrix protein. First, cDNA was synthesized using primer 5'-CACTGGGCACGGTGAGC- 3' (SEQ ID NO: 7), then part of the M1 gene was amplified by running 45 cycles of Light Cycler PCR using primer 5'-CTTCTAACCGAGGTCGAAACGTA-3' (SEQ ID NO: 8) as the reverse primer in the presence of the TaqMan fluorescent probe 5'-6FAM-TCAGGCCCCCTCAAAGCCGA-X-ph (SEQ ID NO: 9). Negative and positive control samples were tested in parallel. The lower limit of detection was determined to be approximately 500 TCID₅₀. Some samples gave inconclusive results, meaning that they gave only a very weak signal (fluorescence < 0.07) after >31 cycles.

### Hemagglutination inhibition assay

Heat-inactivated immune sera from chicken blood samples were tested for HI activity with 1% chicken red blood cells and 4 HAU (hemagglutinating units) of A/Vietnam/1194/04 according to standard methods. Red button formation was scored as evidence of hemagglutination. Antibody titers were expressed as the reciprocal of the highest serum dilution showing HI.

### Results

### Expression, purification, and characterization of the H5 trimers.

In order to express a soluble, trimeric HA ectodomain (sHA³) in mammalian cells, the H5 ectodomain-coding sequence was first cloned into the appropriate expression vector. In the pCD5 vector used, HA-sequence was preceded by a signal peptide-encoding sequence, to allow efficient secretion of the recombinant protein, and followed by sequences coding for the GCN4 isoleucine-zipper trimerizaton motif (Pancera et al., 2005) and the Strep-tagII (Fig. 2A). Expression of the HA ectodomain was achieved by transient transfection of the expression plasmid into HEK cells. Expression and secretion of the HA protein was verified by subjecting cell culture supernatants to gel electrophoresis followed by western blotting using an antibody directed against Strep-tag II (Fig. 2B). The results show that the recombinant HA protein could be readily detected in the cell culture supernatant after transfection of the cells with the expression plasmid, but not after mock transfection. The secreted HA protein was purified in a single step protocol by using Strep-tactin sepharose beads. Protein yields varied between 0.4-1 mg of recombinant protein per 100 ml cell culture medium. After purification of the H5 protein from the cell culture supernatants, the oligomeric state of the H5 protein was analyzed by Superdex200 gel filtration column chromatography (Fig. 2C). The bulk of the HA protein eluted with the velocity of an oligomer, presumably a trimer, while only a minor fraction was found as aggregates in the void volume. The trimeric nature of the H5 oliomer was confirmed using blue-native gel electrophoresis. The biological activity of the purified H5 protein was studied using a solid phase-binding assay with the sialidated blood glycoprotein fetuin. Binding of HA was measured by means of the HRP conjugated to the anti-Strep-tag II antibody as detailed in the Material and Methods section. The H5 preparation demonstrated a concentration dependent binding to fetuin. This binding was sialic acid-dependent, as no binding was observed when the fetuin had been treated with VCNA (Fig. 2D). In conclusion, biologically active, soluble trimeric H5 was efficiently produced in mammalian cells and easily purified.

### Efficacy of sHA³ vaccine against a lethal H5N1 infection in chickens

To assess the protective efficacy of sHA³ in chickens, ten chickens were vaccinated twice (on day 0 and 21) intramuscularly (i.m.) with 10 µg of Stimune-adjuvanted sHA³ and challenged 3 weeks later by intranasal (i.n.)/intratracheal (i.t.) inoculation of a lethal dose of H5N1 A/Vietnam/1194/04 virus. In addition, 10 birds were mock-vaccinated to serve as challenge controls. As shown in Fig. 3, vaccination with 10 ug sHA³ was able to generate complete protection. None of the vaccinated chickens died or showed symptoms indicative of influenza-related disease, whereas all mock-vaccinated chickens succumbed within 2 days. Serological analysis of blood samples showed that all mock-vaccinated chickens had a titer below the detection limit. HI antibody titers increased to a maximum 1024 after an sHA3 boost. The lowest HI titer observed after the boost was 64, which was apparently sufficient to protect the animal against the lethal challenge. Interestingly, 50% of the birds developed HI antibody titers equal to 64 or higher already three weeks after the first vaccination. These results motivated us to estimate the minimal sHA³ dose required to confer protection and to examine whether a single dose with sHA³ could also be protective.

In the second vaccination-challenge experiment, chickens were vaccinated with 10, 2 or 0.4 ug of sHA³ either twice or by a single injection and challenged four weeks later by infection of a lethal dose of A/Vietnam/1194/04 as described above. A booster vaccination with a dose of 0.4 ug of sHA³ was sufficient to protect 90% of the chickens against mortality, while all chickens survived when a booster vaccination of 2 or 10 µg was administered (Fig. 4B). Furthermore the results show that a single vaccination with sHA³ is sufficient to protect the chickens against a lethal infection; only one chicken died when a dose of 2 µg was given, whereas a dose of 10 µg was protective to all birds. Even a single dose of 0.4 µg was still able to protect 60% of the chickens against death. All chickens that received the mock vaccine (PBS) died within 2 days.

We also analyzed whether vaccination with sHA³ decreased or prevented virus shedding. To this end trachea and cloaca swabs were taken from the vaccinated and challenged chickens from the experiment shown in Fig. 4. at day 2, 4 and 7 p.i.. The trachea and cloaca swabs sampled from the same chicken on the same day were pooled and the presence of viral RNA in the pooled swabs was analyzed using a quantitative RT PCR assay detecting the M1 gene. The results are shown in Table 1. Of the chickens that received a booster vaccination with the HA antigen, only 2 birds, both of which had received the lowest amount of antigen, were tested positive. Virus shedding could not be observed in any of the other birds, although 3 swabs gave inconclusive results. Also when the chickens received only one vaccination, it appeared that the majority of the birds did not shed any virus. None of the birds shed the virus at day 4 and 7 post infection after vaccination with 10 µg sHA³. The results show the absence of virus shedding correlated with the birds being protected against a lethal challenge with HPAI H5N1.

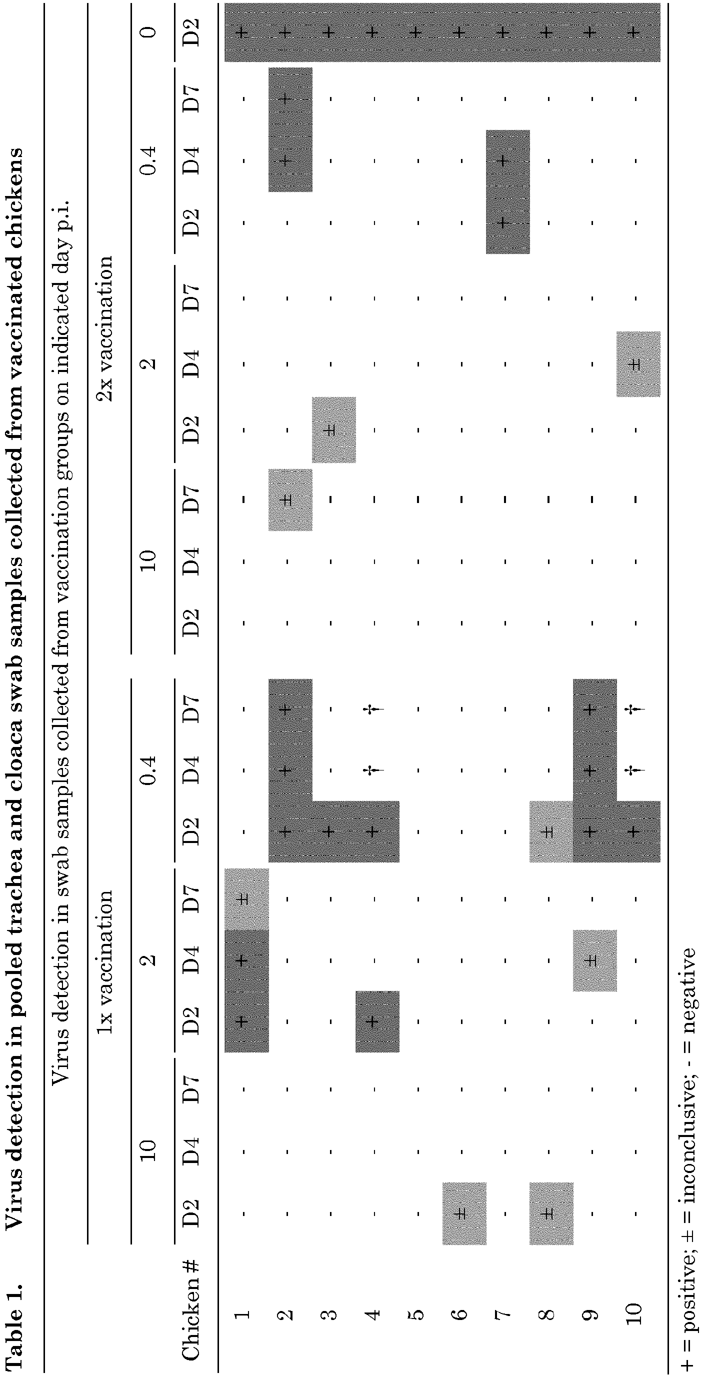

### Example 2

### Materials and methods

### Influenza A viruses

Influenza virus A/Netherlands/602/2009 was isolated from the first case of a laboratory confirmed 2009-A/H1N1 infection in The Netherlands by inoculation of 11-day old embryonated chicken eggs (Munster et al, 2009). Virus stocks of influenza virus A/Netherlands/602/2009 (H1N1) were prepared by infecting confluent Madin-Darby Canine Kidney (MDCK) cells. After cytopathologic changes were complete, culture supernatants were cleared by low speed centrifugation and stored at -70°C. Infectious virus titers were determined in MDCK cells as described previously (Rimmelzwaan et al., 1998). All experiments with these viruses were performed under Bio Safety Level (BSL)-3 conditions.

### Preparation of HA and NA antigens

Human-codon optimized genes encoding the hemagglutinin (HA) ectodomain (a.a. 17-522, figure 10A) and the neuraminidase (NA) head domain (a.a. 75-469, figure 10B) of influenza virus A/California/04/2009(H1N1, UniProt Taxonomy ID: 641501) were synthesized (GenScript) and cloned into a derivative of expression plasmid S1-Ig (Li et al., 2003) for expression in HEK293T cells. The HA gene was flanked by an N-terminal CD5 signal sequence, a C-terminal artificial GCN4 trimerization sequence (GCN4-pII) (Harbury et al., 1993) followed by a *Strep*-tag for affinity purification (IBA GmbH). The NA head domain was flanked by an N-terminal CD5 signal sequence, a double Strep-tag (One-STrEP) sequence (IBA GmbH) and an artificial GCN4 tetramerization domain (GCN4-pLI) (Harbury et al., 1993), respectively.

### Protein expression and purification

HEK293T cells were transfected with the pCD5 expression vectors containing the HA and NA encoding sequences using polyethyleneimine (PEI) in a 1:5 ratio (µg DNA : µg PEI). At 6 h post transfection, the transfection medium was replaced by 293 SFM II expression medium (Invitrogen), supplemented with sodium bicarbonate (3.7 g/liter), glucose (2.0 g/liter), Primatone™ RL-UF (3.0 g/liter, Kerry Bio-Science, Zwijndrecht, The Netherlands), penicillin (100 units/ml), streptomycin (100 µg/ml), glutaMAX (Gibco), and 1.5% DMSO. Tissue culture supernatants were harvested 5-6 days post transfection and HA and NA ectodomains were purified from the culture medium using *Strep*-Tactin affinity chromatography (IBA GmbH). HA and NA protein expression and purification was confirmed by western blotting using a *Strep*-Tactin-HRP conjugate (IBA GmbH) (data not shown) and SDS-PAGE analysis. Oligomerization of the proteins was determined by gel filtration chromatography and by blue-Native-PAGE analysis.

### Ferrets

Healthy young adult outbred female ferrets (*Mustela putorius fur*; between 6 and 12 months old) were supplied by Schimmel, Uddel, The Netherlands. Ferrets were screened for the presence of antibodies against circulating influenza A/H1N1 and A/H3N2 viruses and the swine-origin 2009 A(H1N1) influenza virus [2009A(H1N1) influenza virus] by hemagglutination inhibition assay. Ferrets that were tested negative for these viruses were used in this experiment. An independent animal ethics committee approved the experimental protocol before the start of the experiments.

### Immunizations and infections

Thirty-six seronegative ferrets were divided into six groups of 6 ferrets and vaccinated twice with the following formulations: 3.75 µg trimeric HA + 3.75 µg tetrameric NA in PBS (group 1); 3.75 µg trimeric HA in Iscom Matrix-M (Isconova, Uppsala, Sweden; group 2); 3.75 µg tetrameric NA in Iscom Matrix-M (group 3); 3.75 µg trimeric HA + 3.75 µg tetrameric NA in Iscom Matrix-M (group 4); PBS (group 5); Iscom Matrix-M (group 6). Vaccinations were performed with an interval of 20 days under anesthesia with ketamine in the quadriceps muscles of the hindleg in a total volume of 1ml. Ferrets were housed in groups and received food and water *ad-libitum.* At 32 days after the last vaccination, the animals were anesthesized with ketamine/medetomidine (reversed with atipamezole), weighed and subsequently challenged intratracheally with 1x10⁶TCID₅₀ of influenza A/NL/602/09 (H1N1) in a volume of 3 ml Phosphate Buffered Saline (PBS). After infection, ferrets were monitored three times daily for the development of clinical signs. Before infection and two and four days after infection, nose and throat swabs of each ferret were collected while ferrets were anesthesized with ketamine. Four days after infection, animals were weighed and subsequently killed by exsanguination while under anesthesia with ketamine and medetomidine. Necropsies were performed according to standard procedures.

### Serology

Serum samples were collected before vaccination, at the day of second vaccination (day 20) and at the day of challenge (day 52). Sera were stored at -20°C until use. Sera were tested for the presence of anti-HA antibodies using a hemagglutination inhibition assay (HI-assay) with 1% turkey erythrocytes and for the presence of virus neutralizing antibodies using a micro virus neutralization assay (VN-assay) as described previously (Frank et al., 1980 and Palmer et al., 1975). Sera were tested for the presence of antibodies reactive with influenza A/NL/602/09 (nH1N1). For this purpose, reverse genetics viruses were produced. The titers obtained with these viruses were comparable with those against the wild-type strains (data not shown). Positive control serum specific for influenza A/NL/602/09 (H1N1) was obtained from a ferret infected with this virus (Munster et al., 2009). Sera were also tested for the presence of neuraminidase inhibiting (NI) antibodies using a fetuin-based assay (Lambre et al., 1991). In brief, 96-well Nunc MaxiSorp plates were coated overnight at 4°C with 100 µl of 5 µg/ml fetuin. Sixty-µl volumes of serially diluted serum samples were incubated with an equal volume of S2 cell culture supernatant containing NA (40x diluted in PBS-Ca/Mg [0.901mM/0.493mM], incubated for 30 minutes at 37°C and then 100 µl of the mixture was added to the fetuin-coated plates. After one hour incubation at 37°C, the plates were washed and neuraminidase activity was subsequently measured using a peroxidase-labelled peanut agglutinin (Sigma, Zwijndrecht, The Netherlands) (2.5 µg/ml). Plates were incubated for 1 h at room temperature, washed after which 100 µl of peroxidase substrate (TMB) was added to each well. After 5 minutes, reaction was stopped by the addition of 100 µl of 0.3 M phosphoric acid and OD values were measured at 450nm using an ELISA reader (EL-808 [BioTEK]).

### Virus titers in lungs and in throat and nose swabs

Samples of all lobes of the right lung and of the accessory lobe of infected ferrets were collected and snap frozen on dry ice with ethanol and stored at -70°C until further processing. Lung samples were weighed and subsequently homogenized with a FastPrep-24 (MP Biomedicals, Eindhoven, The Netherlands) in Hank's balanced salt solution containing 0.5% lactalbumin, 10% glycerol, 200 U/ml penicillin, 200 µg/ml streptomycin, 100 U/ml polymyxin B sulfate, 250 µg/ml gentamycin, and 50 U/ml nystatin (ICN Pharmaceuticals, Zoetermeer, The Netherlands) and centrifuged briefly. After collection of nose and throat swabs, swabs were stored at -70°C in the same medium as used to homogenize lung samples. Quadruplicate 10-fold serial dilutions of throat, nose and lung samples were used to infect MDCK cells as described previously (Rimmelzwaan et al., 1998). HA activity of the culture supernatants collected 5 days post inoculation was used as indicator of infection. The titers were calculated according to the Spearman-Karber method and expressed as log TCID₅₀ per gram for lung tissue or per ml for swabs (Karber, 1931).

### Statistical Analysis

Significance among animal groups was analyzed by ANOVA and Tukey test subsequent to ANOVA. Differences were considered significant at P<0.05.

### Results

### Antibody responses induced by immunization with soluble HA and NA

Multimeric, soluble versions of the HA ectodomain (sHA; a.a.7-522) and the neuraminidase head domain (sNA; a.a.75-469) of the pandemic 2009 A(H1N1) influenza virus, expressed in mammalian HEK293T cells and purified from the cell supernatant (Fig.5), were tested for the ability to induce protection against homotypic virus challenge. Ferrets were immunized on day 0 and 20 with sHA and sNA (sHA+sNA), sHA adjuvanted with Iscom Matrix M (IMM; sHA-IMM), sNA-IMM or sHA+sNA-IMM and antibodies induced against the HA and NA proteins by the immunizations were measured in the sera collected at the day of challenge (day 52). Animals vaccinated with sHA-IMM had high hemagglutination inhibition (HI) geometric mean titers (GMT) against the homologous virus (Fig.6A). HI titers were significantly higher in sHA+sNA- IMM immunized animals. Without adjuvant (sHA+sNA) and in the two control groups (IMM or PBS) no HI titer was measurable. Also the neuraminidase inhibition (NI) titration revealed the adjuvant-dependent induction of NA antibodies (Fig.6B). However, in this case no significant enhancement of these titers due to the co-administration of HA was observed. Consistent with the observed HI titers, virus neutralizing (VN) titers were found both in the sHA-IMM and sHA+sNA-IMM vaccinated animals (Fig.6C). Also here the co-administration of sNA antigen in the sHA+sNA-IMM vaccinated animals resulted in an increase in VN titer, going from 1: 202 to 1:468 in the sHA-IMM and sHA+sNA-IMM groups, respectively. Low NI titers were detected in the sera after the first immunization (day 20; data not shown).

### Protection against clinical signs after infection with 2009 A(H1N1) influenza virus

Vaccinated ferrets were challenged intratracheally with 10⁶ TCID₅₀ 2009 A(H1N1) virus 5 weeks after the boost. No clear signs of disease were observed during the immunization period or after the challenge inoculation with 2009 A(H1N1) influenza virus. Yet, loss of body weight became obvious after the inoculation in the PBS and IMM control groups as well as in the non-adjuvanted sHA+sNA vaccine group (Fig.7A). Interestingly, also the animals immunized with sHA-IMM antigen showed similar weight losses while such effects were absent after vaccination with the sNA containing formulations (groups sNA-IMM and sHA+sNA-IMM). The lung weights of the ferrets determined post mortem showed a corresponding tendency of adjuvanted sNA-vaccinated animals having the least disease-related increase due to lung consolidation (Fig.7B).
Histopathological changes were located in multifocal to coalescing lesions with more than 50% of the lungs affected in the unprotected control ferrets inoculated with PBS or adjuvant only (IMM) (Fig.7C). The lesions were characterized by marked inflammatory peribronchiolar lymphocytic infiltrates and occasionally proteinaceous fluid. There was necrosis in the bronchiolar epithelium resulting in cellular debri in the lumen. All these histopathological changes were located in multifocal to coalescing lesions with more than 50% of the lung affected. Clear reduction in histopathological changes was observed in adjuvanted sNA-vaccinated animals (sNA-IMM or sHA+sNA-IMM group). Ferrets that were immunized with sHA-IMM were partially protected from developing pathology showing ∼20% of the lung area being affected.

### Protection of vaccine against virus replication

To measure the effect of vaccination with low doses of 3.75 µg trimeric HA and/or tetrameric NA on the severity of the infection induced by the challenge inoculation with 2009 A(H1N1) influenza virus, virus titers were determined in lungs, throat and nose at day 4 after inoculation. The virus replicated efficiently in the lungs of control ferrets (Fig.8A; PBS and IMM groups) and in the animals immunized with the non-adjuvanted combination of sHA and sNA (sHA+sNA-IMM group), with mean viral titers of approximately 10⁷ - 10⁸. These viral loads were significantly reduced (∼5 log₁₀ units) in the animals immunized with the sHA protein with adjuvant (sHA-IMM group) and in animals co-immunized with sHA and sNA protein with adjuvant (sHA+sNA-IMM group). Mean viral loads were reduced by about 2 log₁₀ in animals immunized with adjuvanted sNA antigen.
High viral loads in the nose were observed both in the control animals (PBS and IMM groups) after inoculation with 2009 A(H1N1) influenza virus. Viral loads in the nose were considerably reduced in the animals immunized with either adjuvanted sHA or sNA or the unadjuvanted sHA+sNA combination. Animals immunized with the adjuvanted combination of sHA and sNA antigens had the highest and significant reduction of nose viral titers (Fig.8B). Differences in throat viral loads of the vaccinated groups compared with control groups were not significant with the exception of the animals vaccinated with adjuvanted combination of sHA and sNA. These ferrets did not have detectable viral titers in the throat.

### Cross-neutralizing antibody responses induced by immunization with soluble HA and NA

The sera of ferrets collected before virus challenge were tested for cross-neutralizing antibodies using HI and NI assays against different other H1N1 influenza strains. Cross-neutralizing antibodies inhibiting hemagglutination against A/NL/386/86, A/NL/25/80 and A/NewYersey/8/76 H1N1 influenza virus were present in the sera of sHA-IMM and sHA+sNA-IMM immunized animals (Fig.9A). Inclusion of sNA in the sHA-containing vaccine resulted in a consistent increase in the hemagglutination inhibition activity towards heterotypic strains, similar as seen for the homologous strain. The relative level of cross-HI antibodies per animal corresponded with that of HI antibodies against the homologous HA. No serum cross-neutralizing activity was detected for A/Swine/shope/1/56, A/Italy/1443/76, A/Iowa/15/30, A/PR/8/34 and IVR/148 influenza H1N1 viruses. Neuraminidase inhibition activity against the sNA of human A/Kentucky/UR06-0258/2007(H1N1) and of avian A/turkey/Turkey/1/2005(H5N1) influenza virus was found in the pooled sera of sNA-IMM and sHA+sNA-IMM immunized animals (Fig.9B).

### Example 3

### Materials and methods

### Cells and viruses

Quail muscle (QM5) cells (Antin & Ordahl, 1991) were cultured in QT35 complete medium (Life Technology) and VERO cells (ATCC CCL-81) were cultured in Dulbecco's modified Eagle's medium (Invitrogen). The media were supplemented with 5% fetal bovine serum and antibiotics. Recombinant fowlpox virus fpEFLT7 (Britton et al., 1996; herein designated FPV-T7), providing stable expression of bacteriophage T7 RNA polymerase in both avian and mammalian cells, was propagated in primary chicken embryo liver cells. Influenza virus A/Vietnam/1194/04 H5N1 (kindly provided by Dr. Alan Hay from the WHO Influenza Centre at the National Institute for Medical Research, London) was propagated in 9-day-embryonated specific pathogen-free (SPF) chicken eggs to produce a virus stock.

### Construction of full-length Newcastle Disease Virus (NDV) cDNAs

Full-length cDNA of NDV Herts, pFL-Herts (de Leeuw et al. 2005), with the unique restriction sites *Asc*I and *Fse*I flanking the F gene, was used as the backbone for the insertion of the H5 HA gene between the P and M genes. To this end, the unique restriction sites *Xba*I and *Pme*I were created in the P-M intergenic region by site-directed mutagenesis. Subsequently, a synthetic linker: GCTCTAGA***TAAGAAAAAATACGGGTAGAA***GTTTAAACGGCGCGCCGG (SEQ ID NO: 10), encoding the sequence of an NDV end-start box (bold) was inserted between the *Xba*I and *Pme*I site (underlined) of the full-length construct.

To modify the polybasic cleavage site of the F0 protein into the monobasic motif (GRQGR↓L; creating FΔPBC), the F gene of Herts was amplified from pFL-Herts^{AF} using the primer pairs pAscI(Herts) (5' TTGGCGCGCCCCAGGTGCAAGATGGGC 3'; SEQ ID NO: 11) together with N043 (5' TTTACTAGTTTACGAAAAGTATTGGATTTGTGCCCC 3'; SEQ ID NO: 12), and N044 (5' GGAGGGAGACAGGGACGCCTTATAGGTGCCATTATCG 3'; SEQ ID NO: 13) together with pFseI(Herts) (5' CCCGATTGAGGGCCGGCCTCCCC 3' SEQ ID NO: 14). To fuse both PCR fragments, a second PCR was performed using the primers pAscI(Herts) and pFseI(Herts). The resulting PCR product was restricted with AscI and FseI and inserted between the AscI and FseI site of pFL-Herts creating pFL-HertsΔPBC.

Viral RNA was extracted from H5N1 A/Vietnam/1194/2004 (NIBRG14) virus using a High Pure Viral RNA kit (Roche). From the extracted viral RNA, cDNA was generated using Superscript II (Invitrogen) and primer CAI085 (5' AGCAAAAGCAGG 3'; SEQ ID NO: 15) matching the non-coding region of each segment. Subsequently the HA gene was PCR amplified using primer pMT036 (AGCTTTGTTTAAAC*A***ATG**GAGAAAATAGTGCTTCTTTTTGC; SEQ ID NO: 16; *Pme*I site underlined) and pMT038 (CCGGCGCGCCGTTTAAAC**TTA**AATGCAAATTCTGCATTGTAAC; SEQ ID NO: 17; *Pme*I site underlined). HA cDNA was subcloned into the pGEM-Teasy plasmid (Promega). This vector was digested with *Pme*I and the HA gene was cloned into pFL-HertsΔPBC generating pFL-HertsAPBC-H5.

To create pFL-HertsΔPBC-H5³, the pCD5-sH5³ expression vector was used (Cornelissen et al. 2010). In this vector, the H5-sequence was preceded by a signal peptide-encoding sequence and followed by sequences coding for the GCN4 isoleucine-zipper trimerizaton motif (Harbury et al. 1993) and the Strep-tag II. The vector was digested with *Pme*I and the liberated insert was ligated with the *Pme*I-digested pFL-HertsΔPBC. The pCD5 vector had been generated such that a *Pme*I restriction site was present upstream of the signal peptide sequence and downstream of the Strep-tag, and the size of the genome still complied with "the rule of six" (de Leeuw et al. 1999) after insertion of the *Pme*I-digest.

All plasmids were generated according to standard procedures (Sambrook et al., 1989). Sequence analysis of modified regions in all constructs generated by PCR was performed to verify that no inadvertent changes had been introduced.

### Recovery of recombinant NDV

Recombinant NDV was generated by reverse genetics using the FPV-T7 RNA polymerase system as described (Peeters et al., 1999). Briefly, VERO cells grown to subconfluency in 6-well dishes were infected with FPV-T7 and transfected with the full-length construct and the helper plasmids expressing NP, P and L. The supernatant was harvested after 6 days of incubation at 37°C, clarified by low-speed centrifugation and filtered through a 0.2 µm filter. The filtrate was used to infect fresh VERO cell cultures. After incubation for 3 days in medium containing 5% allantoic fluid, the supernatant was collected and inoculated (0.2 ml) into the allantoic cavity of 9-to-11 day-embryonated SPF chicken eggs to amplify virus. After 3 days, allantoic fluid was harvested, clarified and aliquoted. Virus recovered from pFL-Herts ΔPBC-H5³ was designated rNDV-H5³ and virus recovered from pFL-Herts ΔPBC-H5 was designated rNDV-HA. Virus titers (TCID₅₀ ml⁻¹) of the harvests were determined in QM5 cells by the end-point dilution method and calculated according to Reed and Münch (Reed and Munch, 1938. A simple method of estimating fifty percent endpoints. Am J Hyg 27: 493-497).

### Intracellular immunostaining

Expression of the HA protein by recombinant NDV in QM5 cells was analyzed by intracellular immunostaining as described (Wensvoort *et al.*, 1986). For this, the cells were permeabilized, fixed with 3% paraformaldehyde and incubated with monoclonal antibody (mAb) 15A6 anti-H5 (1:1000; Santa Cruz). Anti-F mAb 8E12A8C3 (1:5000; CVI, Lelystad) was used for detection of NDV F protein. A rabbit-anti-mouse-HRPO conjugate (1:2000; IBA Germany) was used as the secondary antibody. Mock-infected cell monolayers served as negative controls.

### Production and purification of sH5³

QM5 cells grown to 90% confluency in T75 flasks were infected with rNDV-sH5³ at an MOI of 3. After 72 h of incubation at 37°C, the supernatant was collected (10 ml) and clarified by low-speed centrifugation (10 min at 2500 rpm). The pH of the harvest was adjusted to 7.8 and Avidin was added until a final concentration of 3 µg/ml. After 4 h, recombinant sH5³ protein was purified using Strep-tactin sepharose beads as described by the manufacturer (IBA, Germany). The purification product (15 µl) was subjected to SDS-PAG electrophoreses (12% Bis-Tris) using MOPS running buffer (Invitrogen) and proteins were stained with Blue stain reagent (Thermo Scientific). The sH5³ protein concentration was determined with a nano-drop spectrophotometer and also estimated on gel using a series of bovine serum albumin (BSA) of known amounts as reference.

### Intracerebral pathogenicity index

The standardized Office International des Epizooties (OIE) chicken pathogenicity test was conducted under BSL3 conditions and after approval by the Animal Ethics Committee. Ten one-day-old SPF chickens were inoculated intracerebrally with 0.1 ml of a 1/10 dilution of the egg-grown NDV-HertsAPBC virus and observed for clinical signs every day over a period of 10 days. The birds received a score of 0 if they appeared normal, 1 if they were sick, 2 in case of severe illness and 3 if they were death. The intracerebral pathogenicity index (ICPI) was calculated as the mean score per bird per observation as described in the European Community Council Directive 92/66/EEC (1992). An index of 3.0 means that all birds died within 24 hours.

### Vaccination of chickens and homologous virus challenge

The animal study was conducted under BSL3 conditions and after approval by the Animal Ethics Committee. SPF White Leghorn chickens (Charles River Laboratories, Germany), 6 weeks of age, were used in this study. Ten chickens per group were vaccinated by the intramuscular (i.m.; 0.5 ml) route with 10⁷ TCID₅₀ of vaccine virus.

In addition, one group of 10 chickens was vaccinated i.m. with 5 µg of recombinant sH5³(NDV) protein adjuvated with Stimune (Prionics, Lelystad). Another group of 10 chickens received an equal volume of PBS in Stimune (i.m.) to serve as mock-vaccinated control. Three weeks after the vaccination, all birds were challenged by intranasal (0.1 ml) and intratracheal (0.1 ml) inoculation of in total 10⁵ TCID₅₀ of H5N1 A/Vietnam/1194/04 virus. The chickens were monitored during the next 10 days for signs of disease and mortality. They received a score of 0 if they appeared normal, 1 if they were sick, 2 in case of severe illness and 3 if they were killed or dead, and were inspected twice a day as long as severe illness was scored. Birds that were reluctant to move in response to manipulation or were unable to reach the feeding and water bin were euthanized. The clinical index was calculated as described above for the ICPI. Blood samples were collected at 3 weeks after immunization.

### HI antibody detection

Heat-inactivated immune sera prepared from chicken blood samples were serially diluted in cell culture medium in twofold steps. The samples were tested for hemagglutination inhibition (HI) activity using 1% chicken red blood cells and 4 hemagglutinating units (HAU) of H5N1 A/Vietnam/1194/04. For determination of NDV-specific HI antibodies 8 HAU of Herts/33 virus were used. Red button formation was scored as evidence of hemagglutination. Antibody titers were expressed as the reciprocal of the highest serum dilution showing HI.

### Results

### Virulence of HertsΔPBC virus

The cleavage site of the F0 protein is the major determinant of virulence of NDV (de Leeuw et al, 2005). In velogenic NDV strains, such as Herts/33, the cleavage site typically has a polybasic amino acid motif, which can be cleaved by intracellular proteases allowing the virus to grow in multiple organs. In order to generate attenuated virus, the polybasic cleavage site (PBC) of the F0 gene of pFL-Herts was modified into a monobasic cleavage motif. To confirm the attenuated nature of the virus recovered from this construct, its pathogenicity was tested in vivo. To this end, ten chickens received 0.1 ml of virus by intracerebral injection. Calculation of the clinical scores after 10 days of observation revealed an ICPI of 0.04, demonstrating that modification of the PBC indeed had turned the virus into a lentogenic pathotype.

### Propagation of rNDV-sH5³ and expression of sH5³ protein

rNDV-sH5³ virus could be readily recovered upon transfection of pFL-HertsΔPBC-H5³ into VERO cells and grown up to high titers in embryonated chicken eggs and in the QM5 cell line. The allantoïc fluid of infected eggs yielded a titer of 8.5 and the supernatant of QM5 cells a titer of 7.5 log TCID₅₀ ml⁻¹. Egg-cultured rNDV-HA vaccine virus had a titer of 7.5 log TCID₅₀ ml⁻¹.

To confirm expression of HA, QM5 cells grown in 6-well dishes were infected with egg-cultured rNDV-sH5³ vaccine virus or mock-infected. As controls for protein expression, cells were infected with *wt* NDV (Herts) or rNDV-HA. At 2 days p.i. (post infection), the monolayers were subjected to immunostaining using F- and HA-specific mAbs. As shown in Fig 11B, the monolayer stained positive for F protein after infection with *wt* NDV or chimeric NDV recombinant virus, but not after mock-infection (Fig. 11B). Cell monolayers infected with NDV-H5 and NDV-sH5³, but not *wt* NDV, stained positive for HA. These results demonstrate that HA protein was expressed by NDV-sH5³. Infection with NDV-sH5³ gave a clear but less intense and more diffuse staining pattern compared to that observed in NDV-HA infected cells, presumably because the sH5³ protein does not accumulate in the cells but is secreted.

### Production and purification of NDV-expressed sH5³ protein

sH53 protein was purified from the supernatant of NDV-sH5³-infected QM5 cells by using Strep-tactin beads. The purification product was analyzed by SDS-PAGE followed by Blue staining. As Fig 11C shows, the sH5³(NDV) appeared as a band of ∼70 kDa, migrating somewhat slower than the HEK 293S-produced sH5³ protein. Based on a series of known amounts of BSA that were run in the same gel, the yield of purified sH5³ protein was estimated at 0.6-0.7 mg per 100 ml. The trimeric nature of the H5 oligomer was confirmed using Blue-native gel electrophoresis (Fig. 11D).

### Vaccination of chickens with NDV- sH5³ or sH5³ protein

To examine the immunogenicity of sH5³ (NDV) and the potential of NDV-sH5³ as a live vaccine, ten chickens were vaccinated intramuscularly (i.m.). To examine the protective efficacy of sH5³ protein produced in NDV-sH5³-infected QM5 cells, one group of ten chickens was vaccinated i.m. with Stimune-adjuvanted protein. Another 10 birds were mock-vaccinated to serve as challenge controls. Three weeks after the vaccination, all chickens were challenged with a lethal dose of the homologous H5N1 virus. As expected on basis of the ICPI, none of the chickens showed clinical signs upon vaccination.

The percentage of chickens surviving the infection and clinical scores per group observed after the challenge are shown in Fig. 12A. Intramuscular immunization with NDV-sH5³ or sH5³ protein conferred complete protection. All vaccinated chickens survived and none showed symptoms indicative of influenza-related disease, whereas all mock-vaccinated chickens succumbed within 2 days.

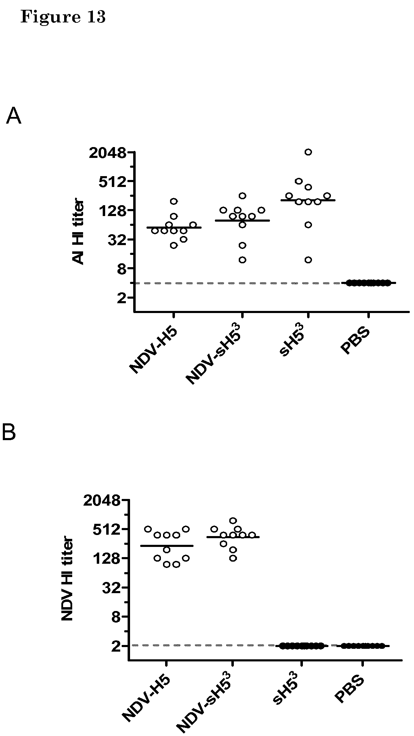

Blood samples were collected 3 weeks after the immunization and assayed for the presence of HI antibodies directed against NDV and influenza virus. The HI assay revealed that i.m. vaccination was able to generate an immune response. HI antibody titers detected after i.m. vaccination with NDV-sH5³ or sH5³ protein reached levels between 12 and 256 (Fig. 13A), comparable to those detected in NDV-HA-vaccinated chickens, which were apparently sufficient to protect all animals against the lethal infection. No HI antibodies were detectable in mock-vaccinated chickens.

All chickens that were vaccinated with rNDV-H5³ showed an immune response to NDV, whereas the chickens that received an i.m. injection of sH5³ only developed influenza HI antibody titers, as expected. NDV HI antibody titers of the NDV-sH53-vaccinated groups reached the same level as the titers of the NDV-HA-vaccinated groups (Fig. 13B). This suggests that NDV-HA and NDV-sH5³ replicated equally well in the chickens. The protection conferred against NDV has not been assessed, but the NDV HI titers are sufficiently high to assume that the vaccination also provides protection against NDV.

### References

Antin, P. B. & Ordahl, C. P. (1991). Isolation and characterization of an avian myogenic cell line. Dev Biol 143, 111-121.
Britton, P., Green, P., Kottier, S., Mawditt, K. L., Penzes, Z., Cavanagh, D. & Skinner, M. A. (1996). Expression of bacteriophage T7 RNA polymerase in avian and mammalian cells by a recombinant fowlpox virus. J Gen Virol 77, 963-970.
Bukreyev, A., Skiadopoulos, M. H., Murphy, B. R. & Collins, P. L. (2006). Nonsegmented negative-strand viruses as vaccine vectors. J Virol 80, 10293-10306.
Chen J, Deng YM. Influenza virus antigenic variation, host antibody production and new approach to control epidemics. Virol J. 2009 Mar 13;6:30.
Colman PM. Influenza virus neuraminidase: structure, antibodies, and inhibitors. Protein Sci. 1994;3(10):1687-96.
Cornelissen, L. A. H. M., de Vries, R. P., de Boer-Luijtze, E. A., Rigter, A., Rottier, P. J. M. & de Haan, C. A. M. (2010). A Single Immunization with Soluble Recombinant Trimeric Hemagglutinin Protects Chickens against Highly Pathogenic Avian Influenza Virus H5N1. PLoS ONE 5, e10645. doi:10.1371.
De Leeuw O, Peeters B. (1999) Complete nucleotide sequence of Newcastle disease virus: evidence for the existence of a new genus within the subfamily Paramyxovirinae. J Gen Virol. 80 (Pt 1):131-6.
De Leeuw, O. S., Koch, G., Hartog, L., Ravenshorst, N. & Peeters, B. P. H. (2005). Virulence of Newcastle disease virus is determined by the cleavage site of the fusion protein and by both the stem region and globular head of the hemagglutinin-neuraminidase protein. J Gen Virol 86, 1759-1769.
DiNapoli, J. M., Kotelkin, A., Yang, L., Elankumaran, S., Murphy, B. R., Samal, S. K., Collins, P. L. & Bukreyev, A. (2007). Newcastle disease virus, a host range-restricted virus, as a vaccine vector for intranasal immunization against emerging pathogens. Proc Natl Acad Sci USA 104, 9788-9793.
Ducatez MF, Webster RG, Webby RJ. Animal influenza epidemiology. Vaccine. 2008 Sep 12;26 Suppl 4:D67-9.
Ekiert DC, Bhabha G, Elsliger MA, Friesen RH, Jongeneelen M, Throsby M, Goudsmit J, Wilson IA. Antibody recognition of a highly conserved influenza virus epitope. Science. 2009;324(5924):246-51.
Frank, A. L., J. Puck, B. J. Hughes, and T. R. Cate. 1980. Microneutralization test for influenza A and B and parainfluenza 1 and 2 viruses that uses continuous cell lines and fresh serum enhancement. J Clin Microbiol 12:426-32.
Gocnik M, Fislová T, Mucha V, Sládková T, Russ G, Kostolansky F, Varecková E. Antibodies induced by the HA2 glycopolypeptide of influenza virus haemagglutinin improve recovery from influenza A virus infection. J Gen Virol. 2008 Apr;89(Pt 4):958-67.
Harbury, P. B., T. Zhang, P. S. Kim, and T. Alber. 1993. A switch between two-, three-, and four-stranded coiled coils in GCN4 leucine zipper mutants. Science 262:1401-7.
Karber, G. 1931. Beitrag zur kollektiven behandlung pharmakologischer reihenversuche Exp. Pathol. Pharmakol. 162:480-483.
Khanna M, Kumar P, Choudhary K, Kumar B, Vijayan VK. Emerging influenza virus: a global threat. J Biosci. 2008 Nov;33(4):475-82.
Kong WP, Hood C, Yang ZY, Wei CJ, Xu L, Garcia-Sastre A, Tumpey TM, Nabel GJ. Protective immunity to lethal challenge of the 1918 pandemic influenza virus by vaccination. Proc Natl Acad Sci U S A. 2006 Oct 24;103(43):15987-91.
Lambre, C. R., H. Terzidis, A. Greffard, and R. G. Webster. 1991. An enzyme-linked lectin assay for sialidase. Clin Chim Acta 198:183-93.
Li, W., M. J. Moore, N. Vasilieva, J. Sui, S. K. Wong, M. A. Berne, M. Somasundaran, J. L. Sullivan, K. Luzuriaga, T. C. Greenough, H. Choe, and M. Farzan. 2003. Angiotensin-converting enzyme 2 is a functional receptor for the SARS coronavirus. Nature 426:450-4.
Lipatov AS, Govorkova EA, Webby RJ, Ozaki H, Peiris M, Guan Y, Poon L, Webster RG. Influenza: emergence and control. J Virol. 2004 Sep;78(17):8951-9.
Munster, V. J., E. de Wit, J. M. van den Brand, S. Herfst, E. J. Schrauwen, T. M. Bestebroer, D. van de Vijver, C. A. Boucher, M. Koopmans, G. F. Rimmelzwaan, T. Kuiken, A. D. Osterhaus, and R. A. Fouchier. 2009. Pathogenesis and transmission of swine-origin 2009 A(H1N1) influenza virus in ferrets. Science 325:481-3.
Niwa H, Yamamura K, Miyazaki J. Efficient selection for high-expression transfectants with a novel eukaryotic vector. Gene. 1991;108(2):193-9.
Palmer D, D. W., Coleman M, Schild G. 1975. Haemagglutination inhibition test. , Atlanta: US Department of Health, Education and Welfare.
Pancera M, Lebowitz J, Schon A, Zhu P, Freire E, Kwong PD, Roux KH, Sodroski J, Wyatt R. Soluble mimetics of human immunodeficiency virus type 1 viral spikes produced by replacement of the native trimerization domain with a heterologous trimerization motif: characterization and ligand binding analysis. J Virol. 2005 Aug;79(15):9954-69.
Peeters, B. P. H., de Leeuw, O. S., Koch, G. & Gielkens, A. L. (1999). Rescue of Newcastle disease virus from cloned cDNA: evidence that cleavability of the fusion protein is a major determinant for virulence. J Virol 73, 5001-5009.
Pouyani T, Seed B. PSGL-1 recognition of P-selectin is controlled by a tyrosine sulfation consensus at the PSGL-1 amino terminus. Cell. 1995;83(2):333-43.
Reeves PJ, Hwa J, Khorana HG. Structure and function in rhodopsin: kinetic studies of retinal binding to purified opsin mutants in defined phospholipid-detergent mixtures serve as probes of the retinal binding pocket. Proc Natl Acad Sci U S A. 1999 Mar 2;96(5):1927-31.
Rimmelzwaan, G. F., M. Baars, E. C. Claas, and A. D. Osterhaus. 1998. Comparison of RNA hybridization, hemagglutination assay, titration of infectious virus and immunofluorescence as methods for monitoring influenza virus replication in vitro. J Virol Methods 74:57-66.
Saelens X, Vanlandschoot P, Martinet W, Maras M, Neirynck S, Contreras R, Fiers W, Jou WM. Protection of mice against a lethal influenza virus challenge after immunization with yeast-derived secreted influenza virus hemagglutinin. Eur J Biochem. 1999 Feb;260(1):166-75.
Sambrook, J., Fritsch, E. F. & Maniatis, T. (1989). Molecular Cloning: a Laboratory Manual, 2nd edn. Cold Spring Harbor, NY: Cold Spring Harbor Laboratory.
Skehel JJ, Wiley DC. Receptor binding and membrane fusion in virus entry: the influenza hemagglutinin. Annu Rev Biochem. 2000;69:531-69.
Song L, Nakaar V, Kavita U, Price A, Huleatt J, Tang J, Jacobs A, Liu G, Huang Y, Desai P, Maksymiuk G, Takahashi V, Umlauf S, Reiserova L, Bell R, Li H, Zhang Y, McDonald WF, Powell TJ, Tussey L. Efficacious recombinant influenza vaccines produced by high yield bacterial expression: a solution to global pandemic and seasonal needs. PLoS One. 2008 May 21;3(5):e2257.
Song L, Zhang Y, Yun NE, Poussard AL, Smith JN, Smith JK, Borisevich V, Linde JJ, Zacks MA, Li H, Kavita U, Reiserova L, Liu X, Dumuren K, Balasubramanian B, Weaver B, Parent J, Umlauf S, Liu G, Huleatt J, Tussey L, Paessler S. Superior efficacy of a recombinant flagellin:H5N1 HA globular head vaccine is determined by the placement of the globular head within flagellin. Vaccine. 2009 Sep 25;27(42):5875-84.
Stevens J, Blixt O, Tumpey TM, Taubenberger JK, Paulson JC, Wilson IA. Structure and receptor specificity of the hemagglutinin from an H5N1 unfluenza virus. Science. 2006;312(5772):404-10
Wei CJ, Xu L, Kong WP, Shi W, Canis K, Stevens J, Yang ZY, Dell A, Haslam SM, Wilson IA, Nabel GJ. Comparative efficacy of neutralizing antibodies elicited by recombinant hemagglutinin proteins from avian H5N1 influenza virus. J Virol. 2008 Jul;82(13):6200-8.
Wensvoort, G., Terpstra, C., Boonstra, J., Bloemraad, M. & Van Zaane, D. (1986). Production of monoclonal antibodies against swine fever virus and their use in laboratory diagnosis. Vet Macrobaol 12, 101-108.
Zeng Q, Langereis MA, van Vliet AL, Huizinga EG, de Groot RJ. Structure of coronavirus hemagglutinin-esterase offers insight into corona and influenza virus evolution. Proc Natl Acad Sci U S A. 2008 Jul 1;105(26):9065-9.

## Claims

1. An immunogenic composition comprising at least one recombinant multimeric ectodomain of an influenza virus protein or part thereof which has a similar immunogenic activity, fused to a streptavidin affinity tag, and a pharmaceutically acceptable diluent, wherein said recombinant multimeric ectodomain of an influenza virus protein or part thereof is selected from the group consisting of a recombinant trimeric influenza hemagglutinin ectodomain or part thereof and a recombinant tetrameric influenza neuraminidase ectodomain or part thereof.

2. The immunogenic composition according to claim 1 whereby trimerization of the recombinant influenza hemagglutinin ectodomain or part thereof which has a similar immunogenic activity, is provided by a GCN4-based trimerization domain.

3. The immunogenic composition according to claim 1 whereby tetramerization of the recombinant influenza neuraminidase ectodomain or part thereof which has a similar immunogenic activity, is provided by a GCN4-based tetramerization domain.

4. The immunogenic composition according to any one of claims 1-3, further comprising a carrier and/or an adjuvant.

5. A vector comprising an expression cassette, comprising:
a) a nucleic acid sequence encoding a signal sequence;
b) a nucleic acid sequence encoding a recombinant ectodomain of an influenza virus protein or part thereof;
c) a nucleic acid sequence encoding a trimerization or tetramerization sequence, and;
d) a nucleic acid sequence encoding a streptavidin affinity tag.

6. An immunogenic composition comprising the vector according to claim 5, and a pharmaceutically acceptable diluent.

7. The immunogenic composition according to claim 6, wherein said vector is a viral vector, such as Newcastle Disease virus derived vector.

8. The immunogenic composition according to any one of claims 1-4, 6 and 7 for use as a vaccine.

9. Use of the vector according to claim 5 for the preparation of an immunogenic composition for eliciting an immune response against an influenza virus.

10. A method for the preparation of an immunogenic composition according to any one of claims 1-4, 6, and 7, comprising:
a) providing the vector according to claim 5;
b) transforming a host cell with said expression vector;
c) culturing said host cell in a culture medium, thereby allowing expression of the recombinant multimeric ectodomain of an influenza virus protein or part thereof which has a similar immunogenic activity, fused to a streptavidin affinity tag;
d) isolating said recombinant multimeric ectodomain of an influenza virus protein or part thereof which has a similar immunogenic activity, fused to a streptavidin affinity tag, thus preparing an immunogenic composition.

11. The method according to claim 10 wherein said recombinant multimeric ectodomain of an influenza virus protein or part thereof which has a similar immunogenic activity, fused to a streptavidin affinity tag is isolated using said streptavidin affinity tag.

12. Use of an immunogenic composition according to any one of claims 1-4, 6 and 7, for the preparation of a prophylactic agent for eliciting an immune response against an influenza virus.

## Patentansprüche

1. Immunogene Zusammensetzung, umfassend wenigstens eine rekombinante multimere Ektodomäne eines Influenzavirusproteins oder einen Teil davon mit einer ähnlichen immunogenen Aktivität, fusioniert an ein Streptavidin-Affinitäts-Tag, und ein pharmazeutisch unbedenkliches Verdünnungsmittel, wobei die rekombinante multimere Ektodomäne eines Influenzavirusproteins bzw. der Teil davon aus der aus einer rekombinanten trimeren Influenza-Hämagglutinin-Ektodomäne oder einem Teil davon und einer rekombinanten tetrameren Influenza-Neuraminidase-Ektodomäne oder einem Teil davon bestehenden Gruppe ausgewählt ist.

2. Immunogene Zusammensetzung nach Anspruch 1, wobei die Trimerisierung der rekombinanten Influenza-Hämagglutinin-Ektodomäne bzw. des Teils davon mit einer ähnlichen immunogenen Aktivität durch eine GCN4-basierte Trimerisierungsdomäne bereitgestellt wird.

3. Immunogene Zusammensetzung nach Anspruch 1, wobei die Tetramerisierung der rekombinanten Influenza-Neuraminidase-Ektodomäne bzw. des Teils davon mit einer ähnlichen immunogenen Aktivität durch eine GCN4-basierte Tetramerisierungsdomäne bereitgestellt wird.

4. Immunogene Zusammensetzung nach einem der Ansprüche 1-3, ferner umfassend einen Träger und/oder ein Adjuvans.

5. Vektor, umfassend eine Expressionskassette, umfassend:
a) eine Nukleinsäuresequenz, die eine Signalsequenz codiert;
b) eine Nukleinsäuresequenz, die eine rekombinante Ektodomäne eines Influenzavirusproteins oder einen Teil davon codiert;
c) eine Nukleinsäuresequenz, die eine Trimerisierungs- oder Tetramerisierungssequenz codiert, und
d) eine Nukleinsäuresequenz, die ein Streptavidin-Affinitäts-Tag codiert.

6. Immunogene Zusammensetzung, umfassend den Vektor nach Anspruch 5 und ein pharmazeutisch unbedenkliches Verdünnungsmittel.

7. Immunogene Zusammensetzung nach Anspruch 6, wobei es sich bei dem Vektor um einen Virusvektor wie einen von Newcastle-Disease-Virus abgeleiteten Vektor handelt.

8. Immunogene Zusammensetzung nach einem der Ansprüche 1-4, 6 und 7 zur Verwendung als Vakzine.

9. Verwendung des Vektors nach Anspruch 5 zur Herstellung einer immunogenen Zusammensetzung, um eine Immunantwort gegen ein Influenzavirus hervorzurufen.

10. Verfahren zur Herstellung einer immunogenen Zusammensetzung nach einem der Ansprüche 1-4, 6 und 7, umfassend:
a) Bereitstellen des Vektors nach Anspruch 5;
b) Transformieren einer Wirtszelle mit dem Expressionsvektor;
c) Kultivieren der Wirtszelle in einem Kulturmedium, was die Expression der rekombinanten multimeren Ektodomäne eines Influenzavirusproteins oder des Teils davon mit einer ähnlichen immunogenen Aktivität, fusioniert an ein Streptavidin-Affinitäts-Tag, gestattet;
d) Isolieren der rekombinanten multimeren Ektodomäne eines Influenzavirusproteins bzw. des Teils davon mit einer ähnlichen immunogenen Aktivität, fusioniert an ein Streptavidin-Affinitäts-Tag, womit eine immunogene Zusammensetzung hergestellt wird.

11. Verfahren nach Anspruch 10, wobei die rekombinante multimere Ektodomäne eines Influenzavirusproteins bzw. der Teil davon mit einer ähnlichen immunogenen Aktivität, fusioniert an ein Streptavidin-Affinitäts-Tag, unter Verwendung des Streptavidin-Affinitäts-Tag isoliert wird.

12. Verwendung einer immunogenen Zusammensetzung nach einem der Ansprüche 1-4, 6 und 7 zur Herstellung eines Prophylaktikums, um eine Immunantwort gegen ein Influenzavirus hervorzurufen.

## Revendications

1. Composition immunogène comprenant au moins un ectodomaine multimérique recombinant d'une protéine de virus de la grippe, ou une partie de celle-ci qui a une activité immunogène semblable, fusionné avec une étiquette d'affinité pour la streptavidine, ainsi qu'un diluant acceptable d'un point de vue pharmaceutique, dans laquelle ledit ectodomaine multimérique recombinant d'une protéine de virus de la grippe, ou une partie de celle-ci, est choisi dans le groupe constitué par un ectodomaine trimérique recombinant d'une hémagglutinine de la grippe, ou une partie de celle-ci, et un ectodomaine tétramérique recombinant d'une neuraminidase de la grippe ou une partie de celle-ci.

2. Composition immunogène, selon la revendication 1, moyennant quoi une trimérisation de l'ectodomaine recombinant d'une hémagglutinine de la grippe, ou une partie de celle-ci qui a une activité immunogène semblable, est fournie par un domaine de trimérisation basé sur GCN4.

3. Composition immunogène selon la revendication 1, moyennant quoi une tétramérisation de l'ectodomaine recombinant d'une neuraminidase de la grippe ou une partie de celle-ci, qui a une activité immunogène semblable, est fournie par un domaine de tétramérisation basé sur GCN4.

4. Composition immunogène, selon l'une quelconque des revendications 1 à 3, comprenant en outre un transporteur et/ou un adjuvant.

5. Vecteur comprenant une cassette d'expression, comprenant :
a) une séquence d'acide nucléique codant pour une séquence signal ;
b) une séquence d'acide nucléique codant pour un ectodomaine recombinant d'une protéine de virus de la grippe ou une partie de celle-ci ;
c) une séquence d'acide nucléique codant pour une séquence de trimérisation ou de tétramérisation, et ;
d) une séquence d'acide nucléique codant pour une étiquette d'affinité pour la streptavidine.

6. Composition immunogène comprenant le vecteur, selon la revendication 5, et un diluant acceptable d'un point de vue pharmaceutique.

7. Composition immunogène selon la revendication 6, dans laquelle ledit vecteur est un vecteur viral, tel qu'un vecteur dérivé du virus de la maladie de Newcastle.

8. Composition immunogène, selon l'une quelconque des revendications 1 à 4, 6 et 7, destinée à être utilisée comme vaccin.

9. Utilisation du vecteur, selon la revendication 5, pour la préparation d'une composition immunogène destinée à provoquer une réponse immunitaire contre un virus de la grippe.

10. Procédé de préparation d'une composition immunogène, selon l'une quelconque des revendications 1 à 4, 6 et 7, comprenant les étapes consistant à :
a) fournir le vecteur selon la revendication 5 ;
b) transformer une cellule hôte avec ledit vecteur d'expression ;
c) cultiver ladite cellule hôte dans un milieu de culture, en permettant qu'ait lieu de ce fait une expression de l'ectodomaine multimérique recombinant d'une protéine de virus de la grippe, ou une partie de celle-ci qui a une activité immunogène semblable, fusionné avec une étiquette d'affinité pour la streptavidine ;
d) isoler ledit ectodomaine multimérique recombinant d'une protéine de virus de la grippe, ou une partie de celle-ci qui a une activité immunogène semblable, fusionné avec une étiquette d'affinité pour la streptavidine, en préparant ainsi une composition immunogène.

11. Procédé selon la revendication 10, dans laquelle ledit ectodomaine multimérique recombinant d'une protéine de virus de la grippe, ou une partie de celle-ci qui a une activité immunogène semblable, fusionné avec une étiquette d'affinité pour la streptavidine, est isolé en utilisant ladite étiquette d'affinité pour la streptavidine.

12. Utilisation d'une composition immunogène, selon l'une quelconque des revendications 1 à 4, 6 et 7, pour la préparation d'un agent prophylactique destiné à provoquer une réponse immunitaire contre un virus de la grippe.
